(19) Europäisches Patentamt
European Patent Office
Office européen des brevets

(11) EP 4 093 861 B1

(12) # EUROPÄISCHE PATENTSCHRIFT

(45) Veröffentlichungstag und Bekanntmachung des Hinweises auf die Patenterteilung:
**25.12.2024 Patentblatt 2024/52**

(21) Anmeldenummer: **21702181.5**

(22) Anmeldetag: **19.01.2021**

(51) Internationale Patentklassifikation (IPC):
***G01N 33/573*** *(2006.01)* ***C12N 9/10*** *(2006.01)*
***C12Q 1/48*** *(2006.01)* ***G01N 33/543*** *(2006.01)*

(52) Gemeinsame Patentklassifikation (CPC):
**C12N 9/13; C12Q 1/48; C12Y 208/02004; C12Y 208/02015; G01N 33/54313; G01N 33/573;**
G01N 2333/91194; G01N 2500/02

(86) Internationale Anmeldenummer:
**PCT/EP2021/051033**

(87) Internationale Veröffentlichungsnummer:
**WO 2021/148388 (29.07.2021 Gazette 2021/30)**

(54) **VERFAHREN ZUM NACHWEIS VON HORMONELL AKTIVEN VERBINDUNGEN, KIT UND DEREN VERWENDUNG**

METHOD FOR THE DETECTION OF HORMONALLY ACTIVE COMPOUNDS, KIT AND THEIR USE

PROCÉDÉ DE DÉTECTION DE COMPOSÉS À ACTION HORMONALE, KIT ET LEUR UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.01.2020 DE 102020101223**
**21.02.2020 DE 102020104629**

(43) Veröffentlichungstag der Anmeldung:
**30.11.2022 Patentblatt 2022/48**

(73) Patentinhaber:
- **Universität Leipzig**
  **04109 Leipzig (DE)**
- **Technische Universität Dresden**
  **01069 Dresden (DE)**

(72) Erfinder:
- **POMPE, Tilo**
  **04109 Leipzig (DE)**
- **RETTKE, David**
  **04109 Leipzig (DE)**
- **OSTERMANN, Kai**
  **01069 Dresden (DE)**
- **DÖRING, Julia**
  **01069 Dresden (DE)**
- **SEUFERT, Florian**
  **04109 Leipzig (DE)**

(74) Vertreter: **Kailuweit & Uhlemann Patentanwälte Partnerschaft mbB**
**Bamberger Straße 49**
**01187 Dresden (DE)**

(56) Entgegenhaltungen:
**EP-A1- 2 752 664**

- **SATO AKIRA ET AL: "Human estrogen sulfotransferase and its related fluorescently labeled decapeptides specifically interact with oxidized low-density lipoprotein", JOURNAL OF PEPTIDE SIENCE, vol. 26, no. 10, 1 October 2020 (2020-10-01), Hoboken, USA, XP055796341, ISSN: 1075-2617, DOI: 10.1002/psc.3274**
- **TAJIK SOMAYEH ET AL: "Recent Advances in Electrochemical Sensors and Biosensors for Detecting Bisphenol A", SENSORS, vol. 20, no. 12, 1 June 2020 (2020-06-01), CH, pages 3364, XP055796924, ISSN: 1424-8220, DOI: 10.3390/s20123364**
- **DU LINGYUN ET AL: "An ultrasensitive detection of 17[beta]-estradiol using a gold nanoparticle-based fluorescence immunoassay", ANALYST, vol. 140, no. 6, 1 January 2015 (2015-01-01), UK, pages 2001 - 2007, XP055796925, ISSN: 0003-2654, DOI: 10.1039/C4AN01952K**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **LIU LANHUA ET AL: "Facile screening of potential xenoestrogens by an estrogen receptor-based reusable optical biosensor", BIOSENSORS AND BIOELECTRONICS, ELSEVIER SCIENCE LTD. UK, AMSTERDAM, NL, vol. 97, 18 May 2017 (2017-05-18), pages 16 - 20, XP085112878, ISSN: 0956-5663, DOI: 10.1016/J.BIOS.2017.05.026**
- **G. B. COLE ET AL: "Specific estrogen sulfotransferase (SULT1E1) substrates and molecular imaging probe candidates", PROCEEDINGS OF THE NATIONAL ACADEMY OF SCIENCES, vol. 107, no. 14, 6 April 2010 (2010-04-06), pages 6222 - 6227, XP055047471, ISSN: 0027-8424, DOI: 10.1073/pnas.0914904107**
- **PUSSAK DANIEL ET AL: "Specific Adhesion of Carbohydrate Hydrogel Particles in Competition with Multivalent Inhibitors Evaluated by AFM", LANGMUIR, vol. 30, no. 21, 3 June 2014 (2014-06-03), US, pages 6142 - 6150, XP055797159, ISSN: 0743-7463, DOI: 10.1021/la5010006**


Bemerkungen:

Das gesamte Dokument mit Referenztabelle(n) und Sequenzliste(n) kann von der Webseite des EPA heruntergeladen werden

## Beschreibung

Die Erfindung betrifft ein Verfahren zum Nachweis von hormonell aktiven Verbindungen mittels immobilisierter Sulfotransferasen und deformierbarer Partikel sowie einen Kit und deren Verwendung zum Nachweis von hormonell aktiven Verbindungen in Lebensmittel-, Waschmittel-, Spülmittel-, Körperpflegemittel-, Kosmetik-, Pharmaka-, Boden-, Gewässer-, Trink- und/oder Abwasserproben.

Xenohormone oder auch endokrine Disruptoren sind hormonell aktive Verbindungen, insbesondere eine heterogene Gruppe von Verbindungen, die auf das Hormonsystem von Organismen wirken. Die hormonell aktiven Verbindungen können eine Gesundheitsschädigung, u. a. die Entstehung von Brust- und Prostatakrebs, Unfruchtbarkeit, Diabetes mellitus, kardiovaskuläre Erkrankungen, Schilddrüsenerkrankungen sowie neurologische, neurodegenerative und psychische Erkrankungen beim Menschen, verursachen.

Aufgrund der Langlebigkeit sowie der weiten Verbreitung hormonell aktiver Verbindungen erfolgt die Aufnahme durch Organismen und der Eintrag in die Umwelt auf unterschiedlichstem Wege. Zum einen können hormonell aktive Inhaltsstoffe aus Verpackungen in Lebensmittel und Getränke übergehen und werden folglich direkt über die Nahrung aufgenommen. Systemische Belastungen treten darüber hinaus über die Verbreitung in Abwässern auf. Konventionelle Kläranlagen halten derartige Verbindungen meist nur unzureichend zurück, sodass entsprechende Verunreinigungen über den Klärschlamm in Böden, Gewässer und das Grund- sowie Trinkwasser gelangen.

Als prominente Vertreter der Xenohormone gelten unter anderem das synthetische Östrogen Ethinylestradiol (EE2) als Hauptbestandteil hormoneller Kontrazeptiva sowie die "Massenchemikalie" Bisphenol A (BPA), welche als Grundstoff in der Synthese von Polycarbonaten und Epoxidharzen Anwendung findet Erstere Verbindung wird nach Einnahme zum überwiegenden Teil über den Urin in nicht-metabolisierter Form ausgeschieden und gilt als besonders abbauresistent. Gelangt EE2 z.B. über Klärschlamm in die Umwelt, reichen die Folgen für aquatische Lebensräume von Fortpflanzungs- und Entwicklungsstörungen der exponierten Organismen, bis hin zur Ausbildung weiblicher Geschlechtsmerkmale bei männlichen Tieren. Gemäß der Richtlinie 2013/39/EU des Europäischen Parlaments und Rates wurde das synthetische Hormon in die erste "Watch-List" potenziell gewässer-gefährdender Schadstoffe aufgenommen (Richtlinie 2013/39/EU des Europäischen Parlaments und Rates). Mittels der Maßnahme soll eine EU-weite Erhebung von Daten zur Abschätzung des von EE2 ausgehenden Risikos ermöglicht werden.

BPA wird unter anderem zur Herstellung von Polycarbonat-Getränkeflaschen wie auch für Beschichtungen von Konservendosen verwendet, wobei die enthaltenen Lebensmittel aufgrund des direkten Kontakts mit der Verbindung belastet sind. Außerdem führen Epoxidharz-Beschichtungen von beispielsweise Haus-Installationsrohren zu Kontaminationen des Trinkwassers. BPA beeinträchtigt dabei die Fruchtbarkeit und ist schädlich für Wasserorganismen (Wassergefährdungsklasse 2). Weiterhin wird BPA als möglicher Auslöser für Frühreife und Verhaltensstörungen diskutiert. Im Januar 2015 wurde die als unbedenklich geltende tägliche Aufnahme (TDI) von der Europäischen Behörde für Lebensmittelsicherheit (EFSA) von zuvor 50 auf 4 μg/kg Körpergewicht gesenkt (EFSA 2015). Ein rechtsverbindlicher Grenzwert für BPA ist in der Trinkwasserverordnung derzeit nicht festgeschrieben. Als Bewertungsgrundlage für Prüfstellen dienen jedoch humantoxikologisch abgeleitete provisorische Trinkwasserhöchstwerte für materialherrührende Stoffe unter die das "Drinking Water Positive List Limit" fällt. Auf Grundlage des TDI-Wertes unter Berücksichtigung eines Unsicherheitsfaktors ergibt sich daraus ein provisorischer Grenzwert von 12 μg/l für Trinkwasser.

Eine wesentliche Voraussetzung sowohl für die Verfolgung der Eintragswege als auch den Schutz der Bevölkerung vor zu hoher Belastung mit Xenohormonen ist eine einfache und umfassend anwendbare Analytik. Zwar existieren zuverlässige Bestimmungsmethoden zum Nachweis von Xenohormonen, jedoch erreichen diese entweder nicht die erforderlichen Nachweisgrenzen und erfordern somit eine umfangreiche Vorbereitung der Proben oder sind in der Anwendung durch labordiagnostische Verfahren wie LC-MS sehr teuer und arbeitsaufwändig.

Die Standardprozedur zur Erfassung von Xenohormon-Kontaminationen in allen Arten von Lebensmitteln und Trinkwasser basiert auf einer aufwändigen Probenvorbereitung mit verschiedenen Extraktions- und Aufreinigungsschritten und dem aufwändigen labordiagnostischen Nachweis mit chromatografischen Verfahren, gekoppelt mit der Massenspektrometrie (LC-MS, GC-MS).

Des Weiteren werden zur Analyse hormonell aktiver Verbindungen, insbesondere Östrogene oder Androgene, in der Umwelt wirkungsbezogene biologische Laborverfahren, wie etwa der Yeast Estrogen Screen (YES)-Test oder der Yeast Androgen Screen (YAS)-Test, eingesetzt. Bei den Tests werden Hefezellen (*Saccharomyces cerevisiae*) verwendet, bei denen die DNA-Sequenz des humanen Östrogen- oder Androgenrezeptors gekoppelt an ein Reportergen (z.B. lacZ kodierend für das Enzym β-Galactosidase) in das Genom integriert wurde. Die Aktivierung des Rezeptors löst eine Kaskade aus, die zur Expression des Reportergenprodukts (z.B. β-Galctosidase) führt, das ein Substrat (z.B. Chlorophenol red-β-D-galactopyranoside, CPRG) umwandelt. Das Substrat führt im Medium zu einer messbaren Farbveränderung von gelb nach rot (Routledge und Sumpter 1996, Sohoni und Sumpter 1998). Ein Nachteil des YES- und YAS-Tests ist der Einsatz gentechnisch veränderter Organismen, welcher streng reguliert und damit labortechnisch äußerst aufwändig ist.

Routledge und Sumpter offenbaren die Untersuchung der östrogenen Aktivität von Tensiden mittels des YES-Tests (Routledge und Sumpter 1996). Im Vergleich wird das natürliche Östrogen 17β-Estradiol untersucht.

Nachtteilig umfassen der YES- und der YAS-Test eine umfangreiche Extraktion bzw. Aufreinigung der Proben und einen Zeitaufwand von zwei bis drei Tagen. Eine Bestimmung von Xenohormonen in wässrigen Umweltproben ist mit den beschriebenen Verfahren aufgrund der geringen Sensitivität des Hefe-Assays nicht möglich.

Bittner et al. beschreiben die Einbettung der Hefezellen von *Saccharomyces cerevisiae* in eine Polymermatrix, insbesondere Gelatine und Agar, zur Herstellung eines Hefe-Assays zum Nachweis von Östrogenen und Androgenen, welcher außerhalb des Labors ohne Vorbereitungszeiten von mehreren Tagen einsetzbar ist (Bittner et al. 2015). Bittner et al. beschreiben die Durchführung des Hefe-Assays zum Nachweis von Testosteron und 17β-Estradiol in 3 h durch Verwendung der immobilisierten Hefezellen.

Alternative Systeme basieren auf der Erzeugung eines Lumineszenzsignals (Purvis et al. 1991).

Beck et al. offenbaren die Verwendung des YES-Tests zur Untersuchung von Östrogenen, Phytoöstrogenen und Xenoöstrogenen mittels GFP (*Green Fluorescent* Protein)-Reporter (Beck et al. 2005).

Bovee et al. beschreiben die Untersuchung von 17β-Testosteron, 5α-Dihydrotestosteron, Methyltrienolon und 17β-Boldenon mittels des YAS-Tests unter Verwendung des Reporters eGFP (*enhanced Green Fluorescent Protein*) (Bovee et al. 2007).

Wolf et al. beschreiben die Untersuchung von Androgenen und Anti-Androgenen mittels des YAS-Tests in *Schizosaccharamyces pompe* und *Saccharomyces cerevisiae* unter Verwendung des Reporters eGFP (*enhanced Green Fluorescent Protein*), wodurch eine Verringerung der Inkubationszeit auf 24 h im Vergleich zu 48 h mit dem β-Galactosidase-Reporter erreicht wird (Wolf et al. 2010).

WO 2014/ 106 665 A1 offenbart ein Verfahren zur Detektion von Analyten, wobei die Immobilisierung eines Analyten an Hydrogelpartikel erfolgt, welche nachfolgend mit einem auf einer Oberfläche immobilisierten Liganden interagieren. Abhängig vom Grad der Interaktion erfolgt eine Deformierung der Hydrogelpartikel durch Anlagerung an die Oberfläche, sodass die Deformierung mittels Reflexionsinterferenzkontrastmikroskopie detektiert werden kann.

WO 02/ 053 713 A2 offenbart Sulfotransferasen, insbesondere Reagenzien und Verfahren zur Regulierung einer humanen Sulfotransferase. Die Reagenzien zur Regulierung der humanen Sulfotransferase werden durch ein Verfahren gewonnen, bei dem eine Testprobe mit der humanen Sulfotransferase in Kontakt gebracht wird, wobei die Sulfotransferase als Fusionsprotein vorliegen kann und auf diese Weise auf einem festen Träger gebunden werden kann.

US 7 288 641 B1 beschreibt Varianten der Sulfontransferase 1 E1-Sequenz sowie die Immobilisierung von SULT1E1 auf einem Substrat. Stjernschantz et al. offenbaren einen Vergleich von muriner und humaner Östrogen-Sulfotransferase (SULT1 E1)-Inhibierung *in vitro* und *in silico* (Stjernschantz et al. 2010). Weiterhin werden 34 verschiedene endokrine Disruptoren, deren Inhibierung der Östrogen-Sulfotransferase sowie deren IC50-Werte beschrieben.

Du et al. offenbaren einen Immunoassay für die Bestimmung von 17β-Estradiol ($E_2$), unter Verwendung von magnetischen, mit anti-$E_2$ Antikörpern beschichteten Mikropartikeln, die als "Capture Probe" dienen, und "double-codified" Gold-Nanopartikeln (DC-AuNPs), modifiziert mit Ziegen-Anti-Kaninchen-Antikörpern und SH-ds-DNA-Biotin, verwendet in Kombination mit Avidin-FITC als Tracer (Du et al. 2015).

Liu et al. beschreiben einen wiederverwendbaren Faser-Biosensor zum Nachweis von 17β-Estradiol ($E_2$), der auf einer kompetitiven Bindung der Probe an den human Östrogenrezeptor (hERα) und der Bindung freien $E_2$ mit Fluorophor-markiertem anti-$E_2$-Antikörper beruht (Liu et al. 2017).

Cole et al. offenbaren die Entwicklung spezifischer Substrate für die Östrogen-Sulfotransferase hSULT1 E1 zur Herstellung von Sonden für den in vivo-Nachweis des Enzyms in Zusammenhang mit menschlichen Krankheiten (Cole et al. 2010).

Pussak et al. beschreiben eine Methode zur Untersuchung von Wechselwirkungen zwischen Molekülen mittels spezifischer Adhäsion von Hydrogelpartikeln, insbesondere den Nachweis von kompetitiven Bindungspartnern bestimmt durch die Deformation des Hydrogelpartikels (Pussak et al. 2014).

Die Aufgabe der Erfindung besteht daher darin, ein einfach handhabbares, kostengünstiges und schnelles Verfahren zum quantitativen Nachweis von hormonell aktiven Verbindungen zur Verfügung zu stellen.

Die Aufgabe wird durch die Merkmale der unabhängigen Ansprüche gelöst. Vorteilhafte Ausgestaltungen sind in den abhängigen Ansprüchen angegeben.

Ein erster Aspekt der Erfindung betrifft ein Verfahren zum Nachweis von hormonell aktiven Verbindungen umfassend die Schritte:

a) Bereitstellen einer Oberfläche mit einem immobilisierten Analytbindungspartner,
wobei der Analytbindungspartner eine Sulfotransferase ausgewählt aus Östrogensulfotransferase hSULT1 E1 (humane Östrogensulfotransferase) oder eine homologe Östrogensulfotransferase umfasst,

b) Kontaktieren des Analytbindungspartners mit einer Probe enthaltend den Analyten,
wobei der Analyt eine hormonell aktive Verbindung umfassend mindestens eine Phenolgruppe ist und mit dem Analytbindungspartner interagiert,

c) Kontaktieren des Analytbindungspartners mit einem Kompetitor,

wobei der Kompetitor eine hormonell aktive Verbindung umfassend mindestens eine Phenolgruppe ist und über eine funktionelle Gruppe an einen deformierbaren Partikel gebunden ist,
wobei der deformierbare Partikel ein Hydrogelpartikel ist und/oder ein Elastizitätsmodul im Bereich von 5 kPa bis 100 kPa aufweist,
wobei der Kompetitor mit dem Analytbindungspartner interagiert,

d) Detektion der an den Analytbindungspartner gebundenen Kompetitoren durch Detektion der Deformierung der Hydrogelpartikel.

Vorteilhaft weist das Enzym humane Östrogensulfotransferase hSULT1E1 eine große Übereinstimmung der Substratspezifität mit dem humanen Östrogenrezeptor auf. Weiterhin vorteilhaft stellt das erfindungsgemäße Verfahren ein preiswertes Nachweisverfahren für hormonell aktive Verbindungen dar.

In Ausführungsformen erfolgt das erfindungsgemäße Verfahren mit einer Reihenfolge der Schritte a), b), c) und d) oder a), c), b) und d), bevorzugt mit einer Reihenfolge der Schritte a), b), c) und d). Vorteilhaft wird durch das Kontaktieren des Analytbindungspartners mit dem Analyten vor dem Kontaktieren mit dem Kompetitor eine schnellere Einstellung des Bindungsgleichgewichts erreicht und damit eine Verkürzung der Kontaktzeiten.

In einer alternativen Ausführungsform erfolgt eine gleichzeitige Kontaktierung des Analytbindungspartners mit dem Analyten und dem Kompetitor.

In Ausführungsformen der Erfindung erfolgt die Kontaktierung des Analytbindungspartners mit dem Analyten vor der Kontaktierung mit dem Partikel für einen Zeitraum von 1 min bis 30 min, vorzugsweise 1 min bis 20 min, besonders bevorzugt 5 min bis 15 min.

Unter dem Begriff "immobilisiert" wird eine Bindung umfassend kovalente, koordinative, metallische, ionische Bindungen, Wasserstoffbrückenbindungen und/oder Van-der-Waals-Wechselwirkungen verstanden. Bevorzugt erfolgt die Immobilisierung durch kovalente oder koordinative Bindung.

Unter dem Begriff "Sulfotransferase" wird ein Enzym verstanden, welches Sulfonsäuregruppen überträgt.

Unter dem Begriff "homologe Östrogensulfotransferase" wird ein Enzym verstanden, welches die gleichen enzymatischen Reaktionen wie die Östrogensulfotransferase hSULT1E1 durchführt und die gleiche Substratspezifität wie hSULT1E1 aufweist.

In Ausführungsformen weist die homologe Östrogensulfotransferase eine Sequenzidentität von mindestens 85%, bevorzugt 95%, besonders bevorzugt 99%; mit der hSULT1E1 nach SEQ ID No. 1 auf.

In Ausführungsformen ist die Sulfotransferase aus der Gruppe umfassend humane Sulfotransferase 1E1 (hSULT1E1), Östrogensulfotransferase-Isoform X1 *(Pan troglodytes),* Sulfotransferase-Familie 1E Östrogen-bevorzugendes Mitglied 1 *(Homo sapiens),* Kette A der Östrogensulfotransferase *(Homo sapiens),* Östrogensulfotransferase *(Pongo abelii*), Östrogensulfotransferase *(Nomascus leucogenys),* Östrogensulfotransferase-Isoform X1 *(Macaca mulatta),* Östrogensulfotransferase-Isoform X2 *(Macaca mulatta),* Östrogensulfotransferase-Isoform X2 *(Aotus nancymaae),* Östrogensulfotransferase *(Cercocebus atys),* Östrogensulfotransferase *(Macaca fascicularis),* Östrogensulfotransferase *(Mandrillus leucophaeus),* Östrogensulfotransferase *(Macaca mulatta),* Östrogensulfotransferase-Isoform X1 *(Macaca nemestrina*), Östrogensulfotransferase *(Piliocolobus tephrosceles),* Östrogensulfotransferase *(Theropithecus gelada),* Östrogensulfotransferase *(Colobus angolensis palliatus),* Östrogensulfotransferase *(Chlorocebus sabaeus),* Östrogensulfotransferase *(Callithrix jacchus),* Östrogensulfotransferase *(Rhinopithecus roxellana),* Östrogensulfotransferase-Isoform X2 (*Cebus capucinus imitator*), Östrogensulfotransferase (*Papio anubis*), Östrogensulfotransferase-Isoform X1 (*Saimiri boliviensis boliviensis*), Östrogensulfotransferase-Isoform X1 *(Aotus nancymaae),* Östrogensulfotransferase-Isoform X2 *(Saimiri boliviensis boliviensis),* Östrogensulfotransferase-Isoform X1 *(Cebus capucinus imitator*) und Östrogensulfotransferase (*Carlito syrichta*) ausgewählt.

In bevorzugten Ausführungsformen ist die Sulfotransferase aus der Gruppe umfassend humane Sulfotransferase 1E1 (hSULT1E1), Östrogensulfotransferase-Isoform X1 *(Pan troglodytes),* Sulfotransferase-Familie 1E Östrogen-bevorzugendes Mitglied 1 *(Homo sapiens),* Kette A der Östrogensulfotransferase *(Homo sapiens),* Östrogensulfotransferase *(Pongo abelii*), Östrogensulfotransferase *(Nomascus leucogenys),* Östrogensulfotransferase-Isoform X1 *(Macaca mulatta),* Östrogensulfotransferase-Isoform X2 *(Macaca mulatta),* Östrogensulfotransferase-Isoform X2 *(Aotus nancymaae),* Östrogensulfotransferase *(Cercocebus atys),* Östrogensulfotransferase *(Macaca fascicularis),* Östrogensulfotransferase *(Mandrillus leucophaeus),* Östrogensulfotransferase *(Macaca mulatta),* Östrogensulfotransferase-Isoform X1 *(Macaca nemestrina*), Östrogensulfotransferase *(Piliocolobus tephrosceles),* Östrogensulfotransferase *(Theropithecus gelada),* Östrogensulfotransferase *(Colobus angolensis palliatus*), Östrogensulfotransferase *(Chlorocebus sabaeus),* Östrogensulfotransferase *(Callithrix jacchus)* und Östrogensulfotransferase *(Rhinopithecus roxellana),* besonders bevorzugt aus der Gruppe umfassend humane Sulfotransferase 1E1 (hSULT1E1), Östrogensulfotransferase-Isoform X1 *(Pan troglodytes),* Sulfotransferase-Familie 1E Östrogen-bevorzugendes Mitglied 1 *(Homo sapiens)* und Kette A der Östrogensulfotransferase *(Homo sapiens);* ausgewählt.

In bevorzugten Ausführungsformen der Erfindung umfasst der Analytbindungspartner die humane Östrogen-Sulfotransferase nach SEQ ID No. 1.

In Ausführungsformen ist die Probe eine Lebensmittel-, Waschmittel-, Spülmittel-, Körperpflegemittel-, Kosmetik-, Pharmaka-, Boden-, Gewässer-, Trink- und/oder Abwasserprobe, bevorzugt eine wässrige Lösung.

Unter dem Begriff "hormonell aktive Verbindung" werden Verbindungen verstanden, welche an Östrogenrezeptoren, bevorzugt Estrogenrezeptor-$\alpha$ oder Estrogenrezeptor-$\beta$, binden.

In Ausführungsformen ist der Analyt eine Verbindung nach Formel (I) oder (II)

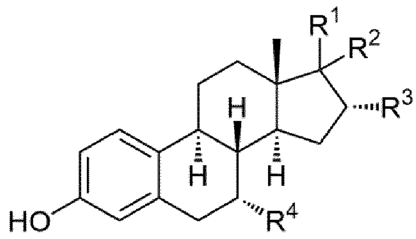 (I) 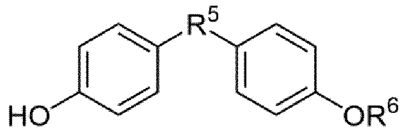 (II),

wobei $R^1$, $R^2$, $R^3$, $R^4$, $R^6$ jeweils unabhängig voneinander ausgewählt ist aus H, funktionellen Gruppen, unsubstituierten oder substituierten C1- bis C15-Alkyl-, Alkenyl- oder Arylgruppen oder Kohlenhydratgruppen, und wobei $R^5$ ausgewählt ist aus unsubstituierten oder substituierten C1- bis C15-Alkyl-, Alkenyl- oder Arylgruppen oder Kohlenhydratgruppen.

Unter dem Begriff "funktionelle Gruppe" wird eine Atomgruppe in einer Verbindung verstanden, welche das Reaktionsverhalten der Verbindung bestimmt. In Ausführungsformen sind funktionelle Gruppen aus funktionellen Gruppen umfassend Heteroatome (O, N, S, P oder Halogene), insbesondere Carbonsäuren, Halogenide, Thiocarbonsäuren, Sulfonsäuren, Thiolester, Sulfoxide, Carbonsäureanhydride, Carbonsäureester, Carbonsäureamide, Nitrile, Aldehyde, Thioaldehyde, Ketone, Thioketone, Oxime, Hydrazone, Alkohole, Thiole, Amine, Imine, Hydrazine, Ether oder Thioether; und funktionellen Gruppen ohne Heteroatome, insbesondere Doppelbindungen, Dreifachbindungen oder Aromaten, ausgewählt.

In Ausführungsformen ist $R^2$, $R^3$, $R^4$ und/oder $R^6$ H.

In weiteren Ausführungsformen ist $R^1$ eine Aldehydgruppe, eine Alkoholgruppe oder eine veresterte Alkoholgruppe.

In weiteren Ausführungsformen umfasst $R^5$ eine substituierte Ethenylgruppe, eine Aryl- oder eine Heteroarylgruppe.

In Ausführungsformen ist $R^1$, $R^2$ oder $R^6$ eine Sulfatgruppe.

In Ausführungsformen ist die Verbindungen nach Formel (II) Bisphenol A .

In alternativen Ausführungsformen ist der Analyt ein Nonylphenol, ein Nonylphenolethoxylat oder ein hydroxylierter (poly-)halogenierter aromatischer Kohlenwasserstoff, insbesondere polychlorierte Dibenzo-p-Dioxine und Dibenzofurane, polybromierte Diphenylether und halogenierte Bisphenol A-Derivate.

Unter dem Begriff "Nonylphenol" werden Verbindungen der allgemeinen Summenformel $C_{15}H_{24}O$ mit einer Molmasse von 220,35 Da verstanden, welche eine Phenolgruppe aufweisen.

Unter dem Begriff "hydroxylierte (poly-)halogenierte aromatische Kohlenwasserstoffe" werden Halogenaromaten verstanden, bei denen mindestens ein Wasserstoffatom durch ein Halogen, insbesondere Fluor, Chlor, Brom und/oder Iod, und mindestens ein Wasserstoffatom durch eine Hydroxylgruppe ersetzt ist.

Kester et al. offenbaren hydroxylierte (poly-)halogenierte aromatische Kohlenwasserstoffe (Kester et al. 2002).

In Ausführungsformen ist der Analyt aus Östrogenen, bevorzugt Ethinylestradiol, Estron, Estradiol, Estriol, Equilin oder $\beta$-Estradiol-17-($\beta$-D-Glucuronid); Östrogenderivaten, hydroxylierten (poly-)halogenierten aromatischen Kohlenwasserstoffen, Nonylphenolen, Bisphenol A, phenolischen Antiöstrogenen, bevorzugt Diethylstilbestrol, 4-Hydroxytamoxifen, Raloxifen oder Fulvestrant; und phenolischen Phytoöstrogenen, bevorzugt Genistein, Daidzein oder Coumestrol; ausgewählt.

Erfindungsgemäß werden unter dem Begriff "Östrogene" hormonell aktive Verbindungen, insbesondere Steroide; verstanden, welche als Grundgerüst Estran mit einem phenolischen A-Ring aufweisen. Vorteilhaft binden Östrogene an den Östrogenrezeptor.

Erfindungsgemäß werden unter dem Begriff "Östrogenderivate" hormonell aktive Verbindungen verstanden, welche als Grundgerüst Estran mit einem phenolischen A-Ring aufweisen sowie mindestens eine Derivatisierung ausgewählt aus einer Alkylierung, insbesondere Methylierung; Glucuronidierung, Halogenierung, Hydroxylierung, Methylierung und Sulfatierung. Vorteilhaft binden Östrogenderivate an den Östrogenrezeptor. Unter dem Begriff "Glucuronidierung" wird eine Bindung einer Glucuronsäure verstanden.

Unter dem Begriff "Antiöstrogene" werden Verbindungen verstanden, welche an den Östrogenrezeptor ohne

Aktivierung binden.

Unter dem Begriff "Phytoöstrogene" werden sekundäre Pflanzenstoffe verstanden, welche an den Östrogenrezeptor binden und daher eine östrogene oder anti-östrogene Wirkung aufweisen.

In Ausführungsformen ist der Analytbindungspartner ein Fusionsprotein. Dabei weist das Fusionsprotein beispielsweise verschiedene Proteindomänen auf, welche unterschiedliche Funktionalitäten aufweisen. Erfindungsgemäß umfasst der Analytbindungspartner eine Proteindomäne, aufweisend eine Analytbindungsstelle. Die Analytbindungsstelle dient der Anbindung des Analyten an den Analytbindungspartner.

Bevorzugt ist der Analytbindungspartner ein Fusionsprotein mit einem Protein-Tag oder Hydrophobin, besonders bevorzugt ein Fusionsprotein mit einem His-Tag. Vorteilhaft wird durch den Protein-Tag oder Hydrophobin eine gerichtete Immobilisierung an der Oberfläche ermöglicht.

In Ausführungsformen weist der Analytbindungspartner die Sequenz SEQ ID No. 2 auf.

In Ausführungsformen der Erfindung ist der deformierbare Partikel ein funktionalisierter Partikel, wobei die Oberfläche des deformierbaren Partikels eine Funktionalisierung, insbesondere funktionelle Gruppen wie Carboxyl- oder Aminogruppen, aufweist. Die Funktionalisierung dient dabei der Immobilisierung des Kompetitors.

In Ausführungsformen ist der deformierbare Partikel ein Hydrogelpartikel, bevorzugt ein Polyethylenglykolhydrogelpartikel.

In Ausführungsformen weist der deformierbare Partikel einen Durchmesser von 10 μm bis 100 μm, besonders bevorzugt ein Durchmesser von etwa 25 μm auf. Der Durchmesser der deformierbaren Partikel kann durch optische Hellfeldmikroskopie bestimmt werden.

Erfindungsgemäß weist der deformierbare Partikel ein Elastizitätsmodul im Bereich von 5 kPa bis 100 kPa, bevorzugt im Bereich von 10 kPa bis 100 kPa, besonders bevorzugt im Bereich von 15 kPa bis 50 kPa; auf. Vorteilhaft wird durch das Elastizitätsmodul im Bereich von 15 kPa bis 50 kPa eine hohe Sensitivität sichergestellt. Weiterhin vorteilhaft wird eine ungleichmäßige Deformation der Partikel ausgeschlossen. Das Elastizitätsmodul der deformierbaren Partikel kann aus Kraft-Abstands-Kurven der Rasterkraftspektroskopie (SFM) bestimmt werden.

In Ausführungsformen der Erfindung weist der deformierbare Partikel eine Carboxy-Funktionalisierung auf. In weiteren Ausführungsformen erfolgt die Synthese und Carboxy-Funktionalisierung der deformierbaren Partikel, insbesondere der Hydrogel-Mikropartikel, gemäß der von Pussak et al. (Pussak *et al.* 2012) beschriebenen Methode via Emulsions- und radikalischer Fällungspolymerisation von Polyethylenglykol-Diacrylamid oder Polyethylenglykol-Diacrylat mit anschließender radikalischer Pfropfung von Acrylsäuremonomeren, Crotonsäuremonomeren oder weiteren Alkenderivaten mit funktionellen Gruppen, wie etwa Aminen zur Einführung der Carboxyl-, Amino- oder anderen funktionellen Gruppen.

In Ausführungsformen erfolgt die Synthese und Carboxy-Funktionalisierung der Partikel mikrofluidisch mittels photoinitiierter radikalischer Vernetzung von Polyethylenglykol-Diacrylamid oder Polyethylenglykol-Diacrylat (bevorzugtes Molekulargewicht im Bereich von 500 Da bis 8.000 Da, besonders bevorzugt etwa 4.000 Da) mit anschließender photoinitiierter radikalischer Pfropfung von Acrylsäuremonomeren zur Einführung der Carboxylgruppen, wobei monodisperse Partikel mit einem Durchmesser im Bereich von 10 μm bis 100 μm, besonders bevorzugt um 25 μm, erzeugt werden.

Erfindungsgemäß ist der Kompetitor ausgebildet zur Interaktion mit dem Analytbindungspartner, wobei der Kompetitor und der Analyt um die Interaktion mit dem Analytbindungspartner konkurrieren. Dadurch wird eine Kompetition zwischen dem freien Analyten in einer Probe und dem partikelgebundenen Kompetitor erzeugt, wobei sich ein Gleichgewicht in Abhängigkeit der Analytkonzentration einstellt. Je mehr freier Analyt vorhanden ist, desto geringer ist die Bindung der partikelgebundenen Kompetitoren an den Analytbindungspartner. Im Ergebnis verringert sich die Kontaktfläche zwischen Partikel und Oberfläche. Im umgekehrten Fall, wenn wenig Analyt in der zu untersuchenden Probe vorhanden ist, erfolgt eine erhöhte Bindung der partikelgebundenen Kompetitoren an den Analytbindungspartner, wobei sich die Kontaktfläche zwischen Partikel und Oberfläche vergrößert. In Ausführungsformen der Erfindung ist der Kompetitor mit dem Analyten identisch.

In Ausführungsformen ist der Kompetitor eine Verbindung nach Formel (I) oder (II)

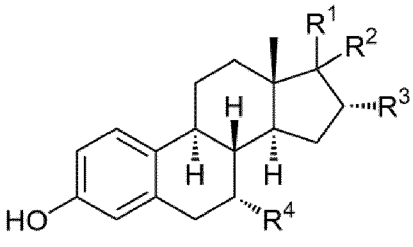 (I)

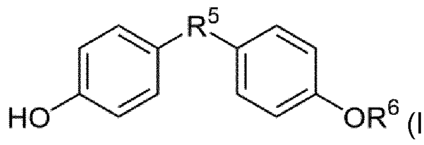 (II),

wobei $R^1$, $R^2$, $R^3$, $R^4$, $R^6$ jeweils unabhängig voneinander ausgewählt ist aus H, funktionellen Gruppen, unsubstituierten oder substituierten C1- bis C15-Alkyl-, Alkenyl- oder Arylgruppen oder Kohlenhydratgruppen,
wobei $R^5$ ausgewählt ist aus unsubstituierten oder substituierten C1- bis C15-Alkyl-, Alkenyl- oder Arylgruppen oder Kohlenhydratgruppen.

In Ausführungsformen ist der Kompetitor aus Östrogenen, bevorzugt Ethinylestradiol, Estron, Estradiol, Estriol, Equilin oder β-Estradiol-17-(β-D-Glucuronid); Östrogenderivaten, hydroxylierten (poly-)halogenierten aromatischen Kohlenwasserstoffen, Nonylphenolen, Bisphenol A, phenolischen Antiöstrogenen, bevorzugt Diethylstilbestrol, 4-Hydroxytamoxifen, Raloxifen oder Fulvestrant; und phenolischen Phytoöstrogenen, bevorzugt Genistein, Daidzein oder Coumestrol; ausgewählt.

In Ausführungsformen der Erfindung ist der Kompetitor aus Östrogen oder einem Östrogenderivat, wie etwa β-Estradiol 17-(β-D-Glucuronid) ausgewählt. In bevorzugten Ausführungsformen ist der Kompetitor β-Estradiol-17-(β-D-Glucuronid).

Erfindungsgemäß ist der Kompetitor über eine funktionelle Gruppe an einen deformierbaren Partikel gebunden. In Ausführungsformen weist der Kompetitor eine funktionelle Gruppe in Position $R^1$, $R^2$, $R^3$, $R^4$, $R^5$ oder $R^6$ auf, bevorzugt in Position $R^1$, $R^2$ oder $R^6$, über die der Kompetitor an den deformierbaren Partikel gebunden ist. Vorteilhaft wird durch die Kopplung in Position $R^1$, $R^2$ oder $R^6$ die Bindung des Kompetitors an das Enzym nicht beeinflusst.

In Ausführungsformen weist der Kompetitor eine Carboxygruppe, eine Ketogruppe, eine Hydroxygruppe oder eine Alkenyl-, Alkinyl- oder Arylgruppe, bevorzugt eine Carboxygruppe, auf. Zweckmäßig erfolgt die Kopplung des Kompetitors an den deformierbaren Partikel über die Carboxygruppe mittels Veresterung oder Amidbindung, über die Ketogruppe mittels reduktiver Aminierung, über die Hydroxygruppe mittels Veresterung oder Kondensationsreaktion mit Etherbildung, über Ethinylgruppe mittels Click-Chemie, insbesondere Kupfer-katalysierte 1,3 dipolare Cycloaddition (Cu-katalysierte Huisgen-Cycloaddition), oder über die Alkenyl-, Alkinyl- oder Arylgruppe mittels Diels-Alder-Reaktion.

In weiteren Ausführungsformen ist der Kompetitor an den deformierbaren Partikel über einen Linker gebunden, wobei der Linker eine Konturlänge im Bereich von 5 Ä bis 200 Å, bevorzugt im Bereich 10 Ä bis 65 Å; und/oder einen Polymerisationsgrad im Bereich von 1 bis 70, bevorzugt im Bereich 3 bis 20; aufweist. Dabei wird der Linker an die funktionalisierte Partikeloberfläche angebunden.

Vorteilhaft ermöglicht ein Linkermolekül die geeignete Immobilisierung des Kompetitors über die Carboxyl- oder die sekundäre Aminogruppe, wobei die Kopplungsgruppe die Funktionalität und Sensitivität des erfindungsgemäßen Verfahrens beeinflusst. Weiterhin vorteilhaft kann über die Länge bzw. den Polymerisationsgrad des Linkers die resultierende Affinität des immobilisierten Kompetitors variiert und somit der Arbeitsbereich des erfindungsgemäßen Verfahrens eingestellt werden.

In Ausführungsformen ist der Linker ausgewählt aus den Gruppen der homo- und heterobifunktionalen Linker und umfasst Ethylendiamin, Oligo- und Polyethylenglykoldiamine, Peptide wie Pentaglycin und Aminosäuren oder einen bifunktionalen Linker mit weiteren Gruppen wie etwa Thiolen oder Aziden. In bevorzugten Ausführungsformen ist der Linker ein Polyethylenglykoldiamin-Linker.

In Ausführungsformen enthalten die Linker Schutzgruppen. In Ausführungsformen der Erfindung ist die Schutzgruppe aus Fluorenylmethoxycarbonyl-, tert-Butyloxycarbonyl- oder tert-Butyl-Schutzgruppen ausgewählt. Vorteilhaft stellen die Schutzgruppen sicher, dass keine unerwünschte Polymerisierung der Linker-Moleküle oder Quervernetzung der deformierbaren Partikel auftritt.

In Ausführungsformen der Erfindung erfolgt die Detektion mittels Quarzkristallmikrowaage (QCM), Oberflächenplasmonenspektroskopie (SPR), Rasterkraftspektroskopie (SFM), Impedanzspektroskopie. oder Reflexionsinterferenzkontrastmikroskopie. Bevorzugt erfolgt die Detektion mittels Reflexionsinterferenzkontrastmikroskopie (RICM). Dabei werden die Kontaktradien $\boldsymbol{a}$ von Partikel und Oberfläche sowie die Partikelradien $\boldsymbol{R_{HGS}}$ mittels einer eigens dafür entwickelten Software automatisiert ermittelt Gemäß Johnson-Kendall-Roberts-Modell (Johnson et al. 1971) stehen diese Größen mit der Adhäsionsenergie $\boldsymbol{W_{adh}}$ in folgendem Zusammenhang:

$$W_{adh} = \frac{\frac{4}{3}a^3\, E_{HGS} \,/\, (1-\nu^2)}{6\pi\, R_{HGS}^2}.$$

Die ermittelte Adhäsionsenergie entspricht der Bindungsdichte der partikelgebundenen Kompetitoren an den Analytbindungspartner und erlaubt die Bestimmung der Analytmenge in der Probe.

In Ausführungsformen des erfindungsgemäßen Verfahrens erfolgt die Detektion mittels Reflexionsinterferenzkontrastmikroskopie. Unter "Reflexionsinterferenzkontrastmikroskopie" wird eine lichtmikroskopische Methode zur Bestimmung von Schichtdicken durch Interferenzmuster verstanden, welche durch an der oberen und unteren Grenzfläche der Schicht reflektierten Lichts entstehen.

In Ausführungsformen der Erfindung ist die Oberfläche transparent, semitransparent oder opak ausgebildet. In weiteren Ausführungsformen ist die Oberfläche zumindest im Bereich von 400 nm bis 600 nm transparent ausgebildet.

In Ausführungsformen der Erfindung ist die Oberfläche planar ausgebildet. Beispielsweise kann die Oberfläche ein Glas- oder Kunststoffformkörper sein, z. B. ein Objektträger, Deckgläschen, Siliziumwafer oder ähnliches. In Ausführungsformen der Erfindung ist die Oberfläche als Well-Platte, insbesondere 16-Well- oder 96-Well-Platte ausgebildet.

Ein weiterer Aspekt der Erfindung betrifft einen Kit umfassend:

i. eine Oberfläche mit einem immobilisierten Analytbindungspartner,
wobei der Analytbindungspartner eine Sulfotransferase ausgewählt aus Östrogen-Sulfotransferase hSULT1E1 (humane Östrogensulfotransferase) oder eine homologe Östrogensulfotransferase umfasst,
ii. zumindest einen deformierbaren Partikel aufweisend einen immobilisierten Kompetitor,

wobei der deformierbare Partikel ein Hydrogelpartikel ist und/oder ein Elastizitätsmodul im Bereich von 5 kPa bis 100 kPa aufweist,
wobei der Kompetitor eine hormonell aktive Verbindung umfassend mindestens eine Phenolgruppe ist und über eine funktionelle Gruppe an den deformierbaren Partikel gebunden ist,
wobei der Kompetitor mit dem Analytbindungspartner interagiert.

In Ausführungsformen des erfindungsgemäßen Kits weist die homologe Östrogensulfotransferase eine Sequenzidentität von mindestens 85%, bevorzugt 95%, besonders bevorzugt 99%; mit der hSULT1E1 nach SEQ ID No. 1 auf.

In Ausführungsformen des erfindungsgemäßen Kits ist die Sulfotransferase aus der Gruppe umfassend humane Sulfotransferase 1E1 (hSULT1E1), Östrogensulfotransferase-Isoform X1 *(Pan troglodytes),* Sulfotransferase-Familie 1E Östrogen-bevorzugendes Mitglied 1 *(Homo sapiens),* Kette A der Östrogensulfotransferase *(Homo sapiens),* Östrogensulfotransferase *(Pongo abelii*), Östrogensulfotransferase *(Nomascus leucogenys),* Östrogensulfotransferase-Isoform X1 *(Macaca mulatta),* Östrogensulfotransferase-Isoform X2 *(Macaca mulatta),* Östrogensulfotransferase-Isoform X2 *(Aotus nancymaae),* Östrogensulfotransferase *(Cercocebus atys),* Östrogensulfotransferase *(Macaca fascicularis),* Östrogensulfotransferase *(Mandrillus leucophaeus),* Östrogensulfotransferase *(Macaca mulatta),* Östrogensulfotransferase-Isoform X1 *(Macaca nemestrina*), Östrogensulfotransferase *(Piliocolobus tephrosceles),* Östrogensulfotransferase *(Theropithecus gelada),* Östrogensulfotransferase *(Colobus angolensis palliatus),* Östrogensulfotransferase *(Chlorocebus sabaeus),* Östrogensulfotransferase *(Callithrix jacchus),* Östrogensulfotransferase *(Rhinopithecus roxellana),* Östrogensulfotransferase-Isoform X2 *(Cebus capucinus imitator*), Östrogensulfotransferase *(Papio anubis),* Östrogensulfotransferase-Isoform X1 *(Saimiri boliviensis boliviensis),* Östrogensulfotransferase-Isoform X1 *(Aotus nancymaae),* Östrogensulfotransferase-Isoform X2 (Saimiri boliviensis boliviensis), Östrogensulfotransferase-Isoform X1 *(Cebus capucinus imitator*) und Östrogensulfotransferase (*Carlito syrichta*) ausgewählt.

In bevorzugten Ausführungsformen ist die Sulfotransferase aus der Gruppe umfassend humane Sulfotransferase 1E1 (hSULT1E1), Östrogensulfotransferase-Isoform X1 *(Pan troglodytes),* Sulfotransferase-Familie 1E Östrogen-bevorzugendes Mitglied 1 *(Homo sapiens),* Kette A der Östrogensulfotransferase *(Homo sapiens),* Östrogensulfotransferase *(Pongo abelii*), Östrogensulfotransferase *(Nomascus leucogenys),* Östrogensulfotransferase-Isoform X1 *(Macaca mulatta),* Östrogensulfotransferase-Isoform X2 *(Macaca mulatta),* Östrogensulfotransferase-Isoform X2 *(Aotus nancymaae),* Östrogensulfotransferase *(Cercocebus atys),* Östrogensulfotransferase *(Macaca fascicularis),* Östrogensulfotransferase (*Mandrillus leucophaeus),* Östrogensulfotransferase *(Macaca mulatta),* Östrogensulfotransferase-Isoform X1 *(Macaca nemestrina*), Östrogensulfotransferase *(Piliocolobus tephrosceles),* Östrogensulfotransferase *(Theropithecus gelada),* Östrogensulfotransferase *(Colobus angolensis palliatus),* Östrogensulfotransferase *(Chlorocebus sabaeus),* Östrogensulfotransferase *(Callithrix jacchus)* und Östrogensulfotransferase *(Rhinopithecus roxellana),* besonders bevorzugt aus der Gruppe umfassend humane Sulfotransferase 1E1 (hSULT1E1), Östrogensulfotransferase-Isoform X1 *(Pan troglodytes),* Sulfotransferase-Familie 1E Östrogen-bevorzugendes Mitglied 1 *(Homo sapiens)* und Kette A der Östrogensulfotransferase *(Homo sapiens);* ausgewählt.

In bevorzugten Ausführungsformen des erfindungsgemäßen Kits umfasst der Analytbindungspartner die humane Östrogen-Sulfotransferase nach SEQ ID No. 1.

In Ausführungsformen des erfindungsgemäßen Kits ist der Kompetitor eine Verbindung nach Formel (I) oder (II)

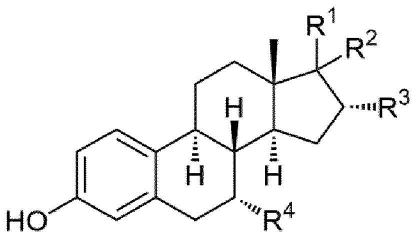
(I)
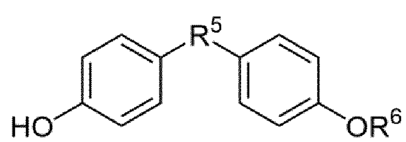
(II),

wobei $R^1$, $R^2$, $R^3$, $R^4$, $R^6$ jeweils unabhängig voneinander ausgewählt ist aus H, funktionellen Gruppen, unsubstituierten oder substituierten C1- bis C15-Alkyl-, Alkenyl- oder Arylgruppen oder Kohlenhydratgruppen,
wobei $R^5$ ausgewählt ist aus unsubstituierten oder substituierten C1- bis C15-Alkyl-, Alkenyl- oder Arylgruppen oder Kohlenhydratgruppen.

In Ausführungsformen des erfindungsgemäßen Kits ist der Kompetitor aus Östrogenen, bevorzugt Ethinylestradiol, Estron, Estradiol, Estriol, Equilin oder β-Estradiol-17-(β-D-Glucuronid); Östrogenderivaten, hydroxylierten (poly-)halogenierten aromatischen Kohlenwasserstoffen, Nonylphenolen, Bisphenol A, phenolischen Antiöstrogenen, bevorzugt Diethylstilbestrol, 4-Hydroxytamoxifen, Raloxifen oder Fulvestrant; und phenolischen Phytoöstrogenen, bevorzugt Genistein, Daidzein oder Coumestrol; ausgewählt.

In Ausführungsformen des erfindungsgemäßen Kits ist der Kompetitor aus Östrogen oder einem Östrogenderivat, wie etwa β-Estradiol 17-(β-D-Glucuronid) ausgewählt. In bevorzugten Ausführungsformen ist der Kompetitor β-Estradiol-17-(β-D-Glucuronid).

Erfindungsgemäß ist der Kompetitor über eine funktionelle Gruppe an einen deformierbaren Partikel gebunden. In Ausführungsformen des erfindungsgemäßen Kits weist der Kompetitor einer funktionellen Gruppe in Position $R^1$, $R^2$, $R^3$, $R^4$, $R^5$ oder $R^6$ auf, bevorzugt in Position $R^1$, $R^2$ oder $R^6$, über die der Kompetitor an den deformierbaren Partikel gebunden ist. Vorteilhaft wird durch die Kopplung in Position $R^1$, $R^2$ oder $R^6$ die Bindung des Kompetitors an das Enzym nicht beeinflusst.

In Ausführungsformen weist der Kompetitor eine Carboxygruppe, eine Ketogruppe, eine Hydroxygruppe oder eine Alkenyl-, Alkinyl- oder Arylgruppe, bevorzugt eine Carboxygruppe, auf. Zweckmäßig erfolgt die Kopplung des Kompetitors an den deformierbaren Partikel über die Carboxygruppe mittels Veresterung oder Amidbindung, über die Ketogruppe mittels reduktiver Aminierung, über die Hydroxygruppe mittels Veresterung oder Kondensationsreaktion mit Etherbildung, über Ethinylgruppe mittels Click-Chemie, insbesondere Kupfer-katalysierte 1,3 dipolare Cycloaddition (Cu-katalysierte Huisgen-Cycloaddition), oder über die Alkenyl-, Alkinyl- oder Arylgruppe mittels Diels-Alder-Reaktion. In weiteren Ausführungsformen des erfindungsgemäßen Kits ist die Oberfläche zumindest im Bereich von 400 nm bis 600 nm transparent ausgebildet.

Ein weiterer Aspekt der Erfindung betrifft die Verwendung des erfindungsgemäßen Verfahrens und/oder des erfindungsgemäßen Kits zum Nachweis von hormonell aktiven Verbindungen in Lebensmittel-, Waschmittel-, Spülmittel-, Körperpflegemittel-, Kosmetik-, Pharmaka-, Boden-, Gewässer-, Trink- und/oder Abwasserproben, bevorzugt in wässrigen Lösungen.

Zur Realisierung der Erfindung ist es auch zweckmäßig, die vorbeschriebenen Ausführungsformen und Merkmale der Ansprüche zu kombinieren.

Nachfolgend soll die Erfindung anhand einiger Ausführungsbeispiele und zugehöriger Figuren eingehender erläutert werden. Die Ausführungsbeispiele sollen dabei die Erfindung beschreiben ohne diese zu beschränken. Der Schutzumfang der Erfindung wird durch die Ansprüche definiert.

Es zeigen die

**Fig. 1** die schematische Darstellung des erfindungsgemäßen Verfahrens beruhend auf der kompetitiven Bindung von Partikel-gebundenem Kompetitor und löslichem Analyt an der Protein-funktionalisierten Oberfläche.
**Fig. 2** die Bindungstaschen des A) Enzyms hSULT1E1 und B) des humanen Östrogenrezeptors mit Estradiol.
**Fig. 3** die Adhäsionsenergie der Partikel beschichtet mit Estradiol-17β-D-Glucuronid in Wechselwirkung mit hSULT1e1-beschichteten Oberflächen (control), Ni-NTA-Oberfläche (NiNTA), und unter Anwesenheit der Analyten Ethinylestradiol (EE), Estron (EST), Testosteron (TE) und Progesteron (PG).

Das Prinzip der Nachweismethode ist in **Fig. 1** dargestellt. Die immobilisierten Enzyme der Oberfläche wechselwirken attraktiv mit dem Partikel-gebundenen Kompetitor, in dessen Folge sich eine charakteristische Kontaktfläche zwischen Oberfläche und Partikel ausprägt. Der gelöste Analyt konkurriert mit dem Kompetitor um die Bindungsstellen auf der Oberfläche. In Abhängigkeit der Konzentration des Analyten verringert sich dadurch die Kontaktfläche. Ab einer bestimmten Konzentration adhärieren die Partikel nicht auf der Oberfläche. Die Bestimmung von Kontakt- und Partikelradius zur Ermittlung der Adhäsionsenergie erfolgt mittels Reflexionsinterferenzkontrastmikroskopie.

Herstellung eines deformierbaren Partikels

Um Mikrogelpartikel mit definierter Größe und Form zu erzeugen, wird die Technik des Tröpfchen-basierten Templatings verwendet. Der Versuchsaufbau erfordert zwei hochpräzise Spritzenpumpen (eine für die Ölphase und eine für die wässrige Phase), eine mikrofluidische Durchflusszelle mit strömungsfokussierender Geometrie (25 μm an der tröpfchenbildenden Düse), ein einfaches Lichtmikroskop und eine Hochgeschwindigkeitskamera für Einzeltropfenbeobachtungen. Sofern nicht anders angegeben, enthält die erste Dispersionsphasenlösung 10 % Massenanteil PEG-Diacrylamid (4-8 kDa), versetzt mit 1 % Massenanteil LAP (Lithiumphenyl-2,4,6-trimethylbenzoylphosphinat), beide gelöst in Reinstwasser. Die kontinuierliche Phase enthält 2% Massenanteil eines Tensids (Perfluorpolyether z.B. Polyhexafluoropropylenoxid, alternativ Polyacrylnitrilbutadienacrylat) das in dem fluorierten Öl HFE 7500 gelöst wird. Nach dem Sammeln der Emulsionen in getrennten Eppendorf-Röhrchen werden die Monomertröpfchen 1 min lang mit UV-Licht zu Mikrogelteilchen vernetzt.

Zur Reinigung der Hydrogelpartikel werden 500 $\mu$l Reinstwasser zu der gesammelten Emulsion gegeben, um genügend wässrige Phase bereitzustellen, in die die Partikel übergehen können. Um die Emulsion aufzubrechen und die hergestellten Mikrokügelchen zu isolieren, werden 250 $\mu$l Perfluoroctanollösung (PFO, 20% (v/v) in HFE 7500) hinzugegeben. Die Emulsion Wasser/PFO-Mischung wird geschüttelt und 30 s bei 1840 x g zentrifugiert. Das Öl wird entfernt und es werden erneut 250 $\mu$l Perfluoroctanollösung zugesetzt. Die Partikelsuspension wird ein weiteres Mal zentrifugiert und die Perfluoroctanollösung entfernt Die so erhaltenen Partikel können nun mittels Crotonsäure funktionalisiert werden.

Ausführungsbeispiel 1: Nachweis von Xenohormonen mittels kompetitiver Bindung

Für die Herstellung einer funktionalisierten Oberfläche wurden 24 Deckgläschen (Menzel-Gläser, ø 32 mm) in ein Teflon-Rack gelegt und für 30 min in einem Ultraschallbad mit destilliertem Wasser belassen. Nach dreimaligem Spülen mit destilliertem Wasser wurden die Gläser weitere 30 min in denaturiertem Ethanol per Ultraschallbad gereinigt. Anschließend wurden die Deckgläschen dreimal mit destilliertem Wasser gespült. Die weitere Reinigung wurde unter Verwendung einer Mischung aus 50 ml $H_2O_2$ (35%), 50 ml 25%iger wässriger $NH_3$-Lösung und 250 ml destilliertem Wasser durchgeführt, was einem Gesamtvolumen von 350 ml entspricht. Die Lösung wurde auf einer Heizplatte auf 60°C erhitzt und das Teflon-Rack mit den Deckgläschen 10 Minuten in der Lösung belassen. Nach zweimaligem Spülen mit destilliertem Wasser wurden die Deckgläser im Stickstoffstrom getrocknet. Zur Einführung von Aminogruppen wurde eine 20 mM 3-Aminopropyltriethoxysilan-Lösung in 9:1 (Vol./Vol.) Isopropanol/destilliertes Wasser hergestellt (225 ml Isopropanol, 25 ml destilliertes Wasser und 1,16 ml APTES). Das Teflon-Rack mit den Deckgläsern wurde anschließend für 120 min in der Lösung belassen, gründlich mit Isopropanol gespült und im Stickstoffstrom getrocknet. Danach wurden die Deckgläser in eine Petrischale gelegt und für60 min bei 120 ° C getempert. Zur weiteren Funktionalisierung mit Carboxygruppen wurden die Deckgläschen für 1 h (Raumtemperatur) in 250 ml einer 240 mM Bernsteinsäureanhydrid-Lösung (6 g in 250 ml) in Tetrahydrofuran (THF) (6 g in 250 ml) belassen, danach zweimal mit THF sowie einmal mit Wasser gewaschen und im Stickstoffstrom getrocknet. Um den Chelator $N_\alpha N_\alpha$-Bis(Carboxymethyl)Lysin ($NTA-NH_2$) zu koppeln, wurden Oberflächen zunächst mit 1,5 ml einer 20 mM 1-Ethyl-3-(3-dimethylaminopropyl)carbodiimid (EDC)- und 50 mM N-Hydroxysulfo-succinimid (NHS)-Lösung in MES (3,834 mg/ml EDC und 5,75 mg/ml NHS, pH 5,0) bedeckt und für 15 min bei Raumtemperatur belassen. Nachdem die Reaktionslösung abgenommen und die Deckgläschen je einmal mit MES-Puffer und zweimal mit HEPES-Puffer (pH = 7,0) gewaschen wurden, wurden 1,5 ml 20 mM $NTA-NH_2$ in HEPES pro Deckgläschen zugegeben und für 2 h bei Raumtemperatur belassen und die Oberflächen nach erfolgter Reaktion einmal mit HEPES und einmal mit destillierten Wasser gespült. Zur Beseitigung von Verunreinigungen in Form von Metallionen wurde auf jedes Deckgläschen 1,5 ml $Na_2EDTA$-Lösung (20 mM, 7,45 mg/ml) in destilliertes Wasser gegeben und 30 min bei Raumtemperatur belassen. Die Oberflächen wurden nun mehrfach mit destilliertem Wasser gewaschen, mit je 1,5 ml einer 20 mM $NiCl_2$-Lösung in Citratpuffer bedeckt, nach 30 min mit destilliertem Wasser gespült und im Stickstoffstrom getrocknet. Durch den Citratpuffer werden unerwünschte Fällungen des Nickels vermieden. Die Ni-NTA-Beschichtung erlaubt die Immobilisierung eines Proteins mit einer Hexahistidin-Sequenz. Hierzu wurden die erfindungsgemäßen Oberflächen zunächst an einen 16-Well-Träger (CS16-CultureWell™, Grace Biolabs) mit selbstklebender Unterseite und einem Volumen von 400 $\mu$l/Well geklebt und die Wells zweimal mit je 250 $\mu$l Beladepuffer (150 mM $Na_2HPO_4$, 10 mM Imidazol, pH 8,0) gewaschen. Anschließend wurden in jedes Well 190 $\mu$l Beladepuffer vorgelegt, 10 $\mu$l einer 100 $\mu$g/ml Hexahistidin-SULT1e1-Lösung pro Well zugegeben, die Oberflächen für 60 min bei Raumtemperatur inkubiert und die Oberflächen abschließend mehrfach mit Beladepuffer gewaschen, um unspezifisch gebundenes Protein zu entfernen.

Zur Einführung von Aminogruppen wurden COOH-funktionalisierte Hydrogelsonden mit einem Durchmesser von 22,5 $\mu$m zunächst mit einer 20 mM EDC- und 50 mM sulfoNHS-Lösung (100 mM HEPES, pH = 7,0) unter Rühren aktiviert. Anschließend wurden 50 $\mu$l einer 50 mM Fluorenylmethoxycarbonyl-NH-PEG-$NH_2$-Lösung zugesetzt und die Kopplung erfolgte über einen Zeitraum von 60 min. Die Schutzgruppe verhindert dabei die Quervernetzung der Partikel. Nach erfolgter Reaktion, wurden die Partikel für 10 min bei 1800 x g zentrifugiert, der Überstand verworfen und die Partikel mehrfach mit HEPES-Puffer (100 mM, pH = 7,0) gespült. Zur Abspaltung der Schutzgruppe wurden die Partikel erneut zentrifugiert, der Überstand verworfen und ein 20 %-iges Piperidin-Wasser-Gemisch zugesetzt. Nach 15-minütiger Reaktion wurden die Partikel wiederum zentrifugiert und mehrfach mit destilliertem Wasser und HEPES-Puffer gespült. Zur weiteren Funktionalisierung wurde zunächst eine 4,1 nM Estradiol-17$\beta$-D-Glucuronid Lösung (950 $\mu$l HEPES, 100 mM, pH = 7,0, 150 $\mu$l Dimethylsulfoxid) präpariert und durch Zusatz von EDC (18,1 mM) und s-NHS (45,4 mM) für 15 min aktiviert. Nach erfolgter Aktivierung konnte das Estradiolderivat durch Zusatz des Gemischs zu den Partikeln und

zweistündiger Reaktion unter Rühren an die Sonden gekoppelt werden. Die Partikel wurden nun erneut mehrfach mit HEPES-Puffer gewaschen. Um mögliche störende Effekte durch verbleibende Aminogruppen zu vermeiden, erfolgte in einem letzten Schritt die Kopplung von Glykolsäure. Hierzu wurde eine 50 mM Glykolsäure/20 mM EDC/50 mM sulfo-NHS-Lösung in HEPES-Puffer präpariert, die COOH-Gruppe für 15 min aktiviert und das Gemisch anschließend den Hydrogelsonden zugesetzt. Nach 2-stündiger Reaktion unter Rühren wurden die Partikel zentrifugiert und mehrfach mit HEPES-Puffer gewaschen.

Nachdem die Oberfläche, beispielsweise eine Glasoberfläche, und die Partikel beschichtet wurden, konnten sie für die Reflexionsinterferenzkontrastmikroskopie (RICM) eingesetzt werden. Hierzu wurden die erfindungsgemäßen Oberflächen an einen 16-Well-Träger (CS16-CultureWell™, Grace Biolabs) mit selbstklebender Unterseite und einem Volumen von 400 μl/Well geklebt.

Anschließend wurden je 200 μl/Well Analytlösung (100 mM HEPES-Puffer pH = 7) zugesetzt. Nach 30-minütiger Inkubation der Oberflächen wurden 10 μl/Well der mit Estradiol-17β-D-Glucuronid-funktionalisierten Hydrogelpartikel hinzugefügt und die Oberflächen nach Sedimentation der Hydrogelpartikel mikroskopiert.

Die Aufnahme der radialen Intensitätsprofile der Hydrogelpartikel auf der funktionalisierten Oberfläche im Reflexionsinterferenzkontrastverfahren erfolgte mittels Inversmikroskopsystem (Olympus IX 73) mit einem 60 x Immersionsobjektiv (Olympus UPlanSAPO 60x Oil Microscope Objective). Aus den aufgenommenen Profilen konnten anschließend Kontaktradien $\boldsymbol{a}$ von Partikel und Oberfläche sowie die Partikelradien $\boldsymbol{R_{HGS}}$ mittels einer eigens dafür entwickelten Software automatisiert ermittelt werden. Gemäß Johnson-Kendall-Roberts-Modell stehen diese Größen mit der Adhäsionsenergie $\boldsymbol{W_{adh}}$ in folgendem Zusammenhang (Johnson et al. 1971):

$$W_{adh} = \frac{\frac{4}{3} a^3 \, E_{HGS} \, / \, (1 - \nu^2)}{6\pi \, R_{HGS}^2}$$

Mit einem Elastizitätsmodul $\boldsymbol{E_{HGS}}$ der Partikel von 15 kPa und einer Poissonzahlv von 0,5 kann somit unter Kenntnis des Partikel- und Kontaktradius die korrespondierende Adhäsionsenergie des Systems bestimmt werden.

Die Ergebnisse der Messungen sind beispielhaft in **Fig. 3** gezeigt. Die Estradiol-17β-D-Glucuronid-funktionalisierten Partikel in Kontakt mit der reinen Ni-NTA-Oberfläche (Negativkontrolle) zeigen lediglich schwache unspezifische Wechselwirkungen mit der Oberfläche, wohingegen die Sonden auf SULT1e1-beschichteten Oberflächen stark adhärieren. Bei Anwesenheit hoher Konzentrationen des Analyten (Ethinylestradiol, Estron) bewegt sich der Wert im Bereich der Negativkontrolle. Dies ist auf die Kompetition um Bindungsstellen der SULT1e1 auf der Oberfläche zwischen freien Ethinylestradiol bzw. Estron in der Analytlösung und an den Hydrogelpartikel gebundenen Estradiol-17β-D-Glucuronid zurückzuführen. Des Weiteren verdeutlicht der vernachlässigbare Einfluss von Androgenen, z.B. Testosteron; und Gestagenen, z.B. Progesteron; auf die Adhäsionsenergie die Selektivität der Methode gegenüber Östrogenen.

**Zitierte Nichtpatentliteratur**

Beck V, Pfitscher A, Jungbauer A (2005) GFP-reporter for a high throughput assay to monitor estrogenic compounds. J Biochem Biophys Methods, 64(1), 19-37.

Bittner M, Jarque S, Hilscherova K (2015) Polymer-immobilized ready-to-use recombinant yeast assays for the detection of endocrine disruptive compounds. Chemosphere, 132, 56-62. Bovee TFH, Helsdingen RJR, Hamers ARM, van Duursen MBM, Nielen MWF, Hoogenboom RLAP (2007) A new highly specific and robust yeast androgen bioassay for the detection of agonists and antagonists. Anal Bioanal Chem, 389(5), 1549-1558.

Cole GB, Keum G, Liu J, Small GW, Satayamurthy N, Keep V, Barrio JR (2010) Specific estrogen sulfotransferase (SULT1E1) Substrates and molecular imaging probe candidates Proceedings of the National Academy of Sciences 107 (14), 6222-6227.

Du L, Ji W, Zhang Y, Zhang C, Liu G, Wang S (2015) An ultrasensitive detection of 17β-estradiol using a gold nanoparticle-based fluorescence immunoassay. Analyst 140 (6), 2001-2007.

European Food Safety Authority (EFSA) Panel on Food Contact Materials, Enzymes, Flavourings and Processing Aids (CEF) (2015) Scientific Opinion on the risks to public health related to the presence of bisphenol A (BPA) in foodstuffs: Executive summary. EFSA Journal, 13(1), 3978.

Johnson KL, Kendall K, Roberts AD (1971) Surface energy and the contact of elastic solids. Proc. R. Soc. Lond. Ser. A - Math. Phys. Sci., 324, 301.

Kester MHA, Bulduk S, van Toor H, Tibboel D, Meinl W, Glatt H, Falany CN, Coughtrie MWH, Schuur AG, Brouwer A, Visser TJ (2002) Potent Inhibition of Estrogen Sulfotransferase by Hydroxylated Metabolites of Polyhalogenated

Aromatic Hydrocarbons Reveals Alternative Mechanism for Estrogenic Activity of Endocrine Disrupters. The Journal of Clinical Endocrinology & Metabolism, 87(3), 1142-1150.
Liu L, Zhou X, Lu Y, Shan D, Xu B, He M, Shi H, Qian Y (2017) Facile screening of potential xenoestrogens by an estrogen receptor-based reusable optical biosensor. Biosensors and Bioelectronics 97, 16-20.
Purvis IJ, Chotai D, Dykes CW, Lubahn DB, French FS, Wilson EM, Hobden AN (1991) An androgen-inducible expression system for Saccharomyces cerevisiae. Gene, 106, 35-42. Pussak D, Behra M, Schmidt S, Hartmann L (2012) Soft Matter, 8, 1664.
Pussak D, Ponader D, Mosca S, Pompe T, Hartmann L, Schmidt S (2014) Specific Adhesion of Carbohydrate Hydrogel Particles in Competition with Multivalent Inhibitors Evaluated by AFM. Langmuir 30 (21), 6142-6150.
Richtlinie 2013/39/EU des Europäischen Parlaments und Rates (2013) RICHTLINIE 2013/39/EU DES EUROPÄISCHEN PARLAMENTS UND DES RATES vom 12. August 2013 zur Änderung der Richtlinien 2000/60/EG und 2008/105/EG in Bezug auf prioritäre Stoffe im Bereich der Wasserpolitik. Amtsblatt der Europäischen Union, L 226/1-L 226/17.
Routledge EJ, Sumpter JP (1996) Estrogenic activity of surfactants and some of their degradation products assessed using a recombinant yeast screen. Environmental Toxicology and Chemistry, 15 (3), 241-248.
Sohoni P, Sumpter JP (1998) Several environmental oestrogens are also anti-androgens. Endocrinol, 158, 327-339.
Stjernschantz E, Reinen J, Meinl W, George BJ, Glatt H, Vermeulen NPE, Oostenbrink C (2010) Comparison of murine and human estrogen sulfotransferase inhibition in vitro and in silico - Implications for differences in activity, subunit dimerization and substrate inhibition. Mol. Cell. Endocrinol. 317, 127-140.
Wolf S, Rataj F, Zierau O, Ostermann K, Diel P, Parr MK, Vollmer G, Rödel G (2010) A novel combined approach to detect androgenic activities with yeast based assays in Schizosaccharomyces pombe and Saccharomyces cerevisiae. Toxicology Letters, 199, 410-415.

## Patentansprüche

1. Verfahren zum Nachweis von hormonell aktiven Verbindungen umfassend die Schritte:

   a) Bereitstellen einer Oberfläche mit einem immobilisierten Analytbindungspartner,
   wobei der Analytbindungspartner eine Sulfotransferase ausgewählt aus Östrogensulfotransferase hSULT1E1 (humane Östrogensulfotransferase) oder eine homologe Östrogensulfotransferase umfasst,
   b) Kontaktieren des Analytbindungspartners mit einer Probe enthaltend den Analyten,
   wobei der Analyt eine hormonell aktive Verbindung umfassend mindestens eine Phenolgruppe ist und mit dem Analytbindungspartner interagiert,
   c) Kontaktieren des Analytbindungspartners mit einem Kompetitor,

      wobei der Kompetitor eine hormonell aktive Verbindung umfassend mindestens eine Phenolgruppe ist und über eine funktionelle Gruppe an einen deformierbaren Partikel gebunden ist,
      wobei der deformierbare Partikel ein Hydrogelpartikel ist und/oder ein Elastizitätsmodul im Bereich von 5 kPa bis 100 kPa aufweist,
      wobei der Kompetitor mit dem Analytbindungspartner interagiert,

   d) Detektion der an den Analytbindungspartner gebundenen Kompetitoren durch Detektion der Deformierung der Hydrogelpartikel.

2. Verfahren nach Anspruch 1, **dadurch gekennzeichnet, dass** die homologe Östrogensulfotransferase eine Sequenzidentität von mindestens 85% mit der hSULT1E1 nach SEQ ID No. 1 aufweist.

3. Verfahren nach Anspruch 1 oder 2, **dadurch gekennzeichnet, dass** der Analyt eine Verbindung nach Formel (I) oder (II) ist

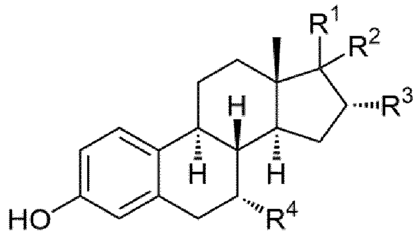 (I) 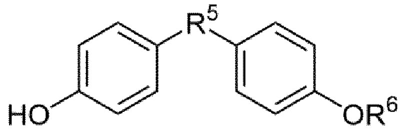 (II),

wobei $R^1$, $R^2$, $R^3$, $R^4$, $R^6$ jeweils unabhängig voneinander ausgewählt sind aus H, funktionellen Gruppen, unsubstituierten oder substituierten C1- bis C15-Alkyl-, Alkenyl- oder Arylgruppen oder Kohlenhydratgruppen, wobei $R^5$ ausgewählt ist aus unsubstituierten oder substituierten C1- bis C15-Alkyl-, Alkenyl- oder Arylgruppen oder Kohlenhydratgruppen.

4. Verfahren nach einem der Ansprüche 1 bis 3, **dadurch gekennzeichnet, dass** der Analyt ausgewählt ist aus Östrogenen, Östrogenderivaten, hydroxylierten halogenierten aromatischen Kohlenwasserstoffen, Nonylphenolen, Bisphenol A, phenolischen Antiöstrogenen und phenolischen Phytoöstrogenen.

5. Verfahren nach einem der Ansprüche 1 bis 4, **dadurch gekennzeichnet, dass** der Analytbindungspartner ein Fusionsprotein ist, bevorzugt ein Fusionsprotein mit einem Protein-Tag oder Hydrophobin.

6. Verfahren nach einem der vorhergehenden Ansprüche, **dadurch gekennzeichnet, dass** der Kompetitor eine Verbindung nach Formel (I) oder (II) ist

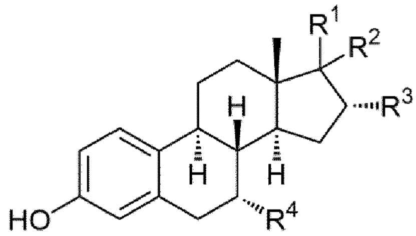 (I) 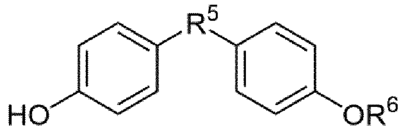 (II),

wobei $R^1$, $R^2$, $R^3$, $R^4$, $R^6$ jeweils unabhängig voneinander ausgewählt ist aus H, funktionellen Gruppen, unsubstituierten oder substituierten C1- bis C15-Alkyl-, Alkenyl- oder Arylgruppen oder Kohlenhydratgruppen,
wobei $R^5$ ausgewählt ist aus unsubstituierten oder substituierten C1- bis C15-Alkyl-, Alkenyl- oder Arylgruppen oder Kohlenhydratgruppen,
wobei der Kompetitor eine funktionelle Gruppe in Position $R^1$, $R^2$ oder $R^6$ aufweist, über die der Kompetitor an den deformierbaren Partikel gebunden ist

7. Verfahren nach einem der vorhergehenden Ansprüche, **dadurch gekennzeichnet, dass** der Kompetitor β-Estradiol-17-(β-D-Glucuronid) ist.

8. Verfahren nach einem der vorhergehenden Ansprüche, **dadurch gekennzeichnet, dass** der Kompetitor an den deformierbaren Partikel über einen Linker gebunden ist, wobei der Linker eine Konturlänge im Bereich von 5 Å bis 200 Å und/oder einen Polymerisationsgrad im Bereich von 1 bis 70 aufweist.

9. Verfahren nach einem der vorhergehenden Ansprüche, **dadurch gekennzeichnet**, dassdie Detektion mittels Reflexionsinterferenzkontrastmikroskopie erfolgt.

10. Verfahren nach einem der vorhergehenden Ansprüche, **dadurch gekennzeichnet, dass** die Oberfläche zumindest im Bereich von 400 nm bis 600 nm transparent ausgebildet ist.

11. Kit umfassend:

i. eine Oberfläche mit einem immobilisierten Analytbindungspartner,
wobei der Analytbindungspartner eine Sulfotransferase ausgewählt aus Östrogensulfotransferase hSULT1E1 (humane Östrogensulfotransferase) oder eine homologe Östrogensulfotransferase umfasst,

ii. zumindest einen deformierbaren Partikel aufweisend einen immobilisierten Kompetitor,

wobei der deformierbare Partikel ein Hydrogelpartikel ist und/oder ein Elastizitätsmodul im Bereich von 5 kPa bis 100 kPa aufweist,
wobei der Kompetitor eine hormonell aktive Verbindung umfassend mindestens eine Phenolgruppe ist und über eine funktionelle Gruppe an den deformierbaren Partikel gebunden ist,
wobei der Kompetitor mit dem Analytbindungspartner interagiert.

**12.** Kit nach Anspruch 11, **dadurch gekennzeichnet, dass** die homologe Östrogensulfotransferase eine Sequenzidentität von mindestens 85% mit der hSULT1E1 nach SEQ ID No. 1 aufweist.

**13.** Kit nach Anspruch 11 oder 12, **dadurch gekennzeichnet, dass** der Kompetitor eine Verbindung nach Formel (I) oder (II) ist

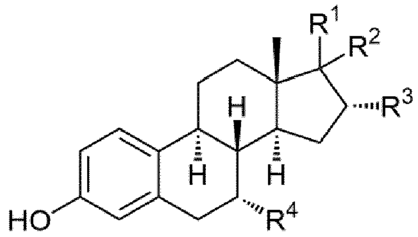 (I) 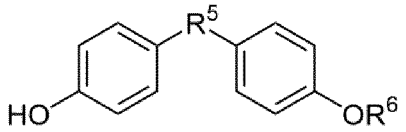 (II),

wobei $R^1$, $R^2$, $R^3$, $R^4$, $R^6$ jeweils unabhängig voneinander ausgewählt sind aus H, funktionellen Gruppen, unsubstituierten oder substituierten C1- bis C15-Alkyl-, Alkenyl- oder Arylgruppen oder Kohlenhydratgruppen,
wobei $R^5$ ausgewählt ist aus unsubstituierten oder substituierten C1- bis C15-Alkyl-, Alkenyl- oder Arylgruppen oder Kohlenhydratgruppen,
wobei der Kompetitor eine funktionelle Gruppe in Position $R^1$, $R^2$ oder $R^6$ aufweist, über die der Kompetitor an den deformierbaren Partikel gebunden ist.

**14.** Verwendung eines Verfahrens nach einem der Ansprüche 1 bis 10 und/oder eines Kits nach einem der Ansprüche 11 bis 13 zum Nachweis von hormonell aktiven Verbindungen in Lebensmittel-, Waschmittel-, Spülmittel-, Körperpflegemittel-, Kosmetik-, Pharmaka-, Boden-, Gewässer-, Trink- und/oder Abwasserproben.

## Claims

**1.** Method for the detection of hormonally active compounds comprising the steps:

a) Providing a surface with an immobilized analyte binding partner, wherein the analyte binding partner comprises a sulfotransferase selected from estrogen sulfotransferase hSULT1E1 (human estrogen sulfotransferase) or a homologous estrogen sulfotransferase,
b) Contacting the analyte binding partner with a sample containing the analyte, wherein the analyte is a hormonally active compound comprising at least one phenol group and interacts with the analyte binding partner,
c) Contacting the analyte binding partner with a competitor,

wherein the competitor is a hormonally active compound comprising at least one phenol group and is bound to a deformable particle via a functional group, wherein the deformable particle is a hydrogel particle and/or has a Young's modulus in the range of 5 kPa to 100 kPa,
wherein the competitor interacts with the analyte binding partner,

d) Detection of competitors bound to the analyte binding partner by detecting the deformation of the hydrogel particles.

**2.** Method according to claim 1, **characterized in that** the homologous estrogen sulfotransferase has a sequence identity of at least 85% with hSULT1E1 according to SEQ ID No. 1.

**3.** Method according to claim 1 or 2, **characterized in that** the analyte is a compound according to formula (I) or (II)

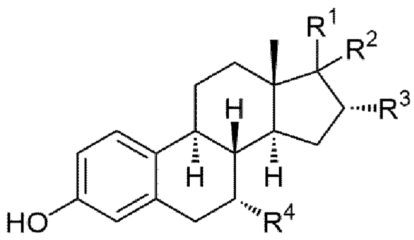 (I) 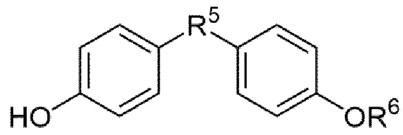 (II),

wherein $R^1$, $R^2$, $R^3$, $R^4$, $R^6$ are each independently selected from H, functional groups, unsubstituted or substituted C1 to C15 alkyl, alkenyl or aryl groups or carbohydrate groups, wherein $R^5$ is selected from unsubstituted or substituted C1 to C15 alkyl, alkenyl or aryl groups or carbohydrate groups.

4. Method according to one of claims 1 to 3 , **characterized in that** the analyte is selected from estrogens, estrogen derivatives, hydroxylated halogenated aromatic hydrocarbons, nonylphenols , bisphenol A, phenolic antiestrogens and phenolic phytoestrogens.

5. Method according to one of claims 1 to 4, **characterized in that** the analyte binding partner is a fusion protein, preferably a fusion protein with a protein tag or hydrophobin.

6. Method according to one of the preceding claims, **characterized in that** the competitor is a compound of formula (I) or (II)

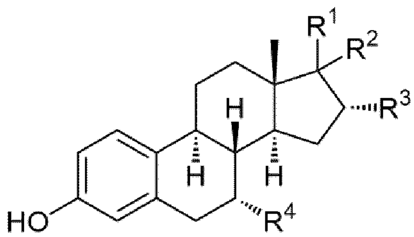 (I) 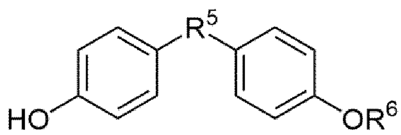 (II),

wherein $R^1$, $R^2$, $R^3$, $R^4$, $R^6$ are each independently selected from H, functional groups, unsubstituted or substituted C1 to C15 alkyl, alkenyl or aryl groups or carbohydrate groups, wherein $R^5$ is selected from unsubstituted or substituted C1 to C15 alkyl, alkenyl or aryl groups or carbohydrate groups,
wherein the competitor has a functional group in position $R^1$ , $R^2$ or $R^6$, via which the competitor is bound to the deformable particle.

7. Method according to one of the preceding claims, **characterized in that** the competitor is β-estradiol-17-(β-D-glucuronide).

8. Method according to one of the preceding claims, **characterized in that** the competitor is bound to the deformable particle via a linker, wherein the linker has a contour length in the range of 5 Å to 200 Å and/or a degree of polymerization in the range of 1 to 70.

9. Method according to one of the preceding claims, **characterized in that** the detection is carried out by means of reflection interference contrast microscopy.

10. Method according to one of the preceding claims, **characterized in that** the surface is transparent at least in the range of 400 nm to 600 nm.

11. Kit includes:

i. a surface with an immobilized analyte binding partner,
wherein the analyte binding partner comprises a sulfotransferase selected from estrogen sulfotransferase hSULT1E1 (human estrogen sulfotransferase ) or a homologous estrogen sulfotransferase,
ii. at least one deformable particle comprising an immobilized competitor,

wherein the deformable particle is a hydrogel particle and/or has a Young's modulus in the range of 5 kPa to 100 kPa,
wherein the competitor is a hormonally active compound comprising at least one phenol group and is bound to the deformable particle via a functional group, wherein the competitor interacts with the analyte binding partner.

**12.** Kit according to claim 11, **characterized in that** the homologous estrogen sulfotransferase has a sequence identity of at least 85% with hSULT1E1 according to SEQ ID No. 1.

**13.** Kit according to claim 11 or 12, **characterized in that** the competitor is a compound according to formula (I) or (II)

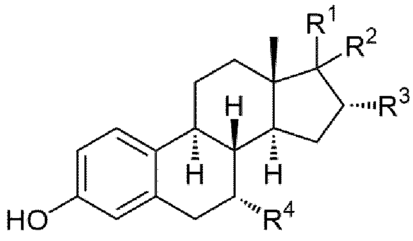
(I)
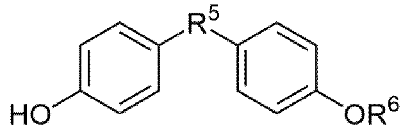
(II),

wherein $R^1$, $R^2$, $R^3$, $R^4$, $R^6$ are each independently selected from H, functional groups, unsubstituted or substituted C1 to C15 alkyl, alkenyl or aryl groups or carbohydrate groups, wherein $R^5$ is selected from unsubstituted or substituted C1 to C15 alkyl, alkenyl or aryl groups or carbohydrate groups,
wherein the competitor has a functional group in position $R^1$ , $R^2$ or $R^6$, via which the competitor is bound to the deformable particle.

**14.** Use of a method according to one of claims 1 to 10 and/or a kit according to one of claims 11 to 13 for the detection of hormonally active compounds in food, detergent, dishwashing detergent, personal care product, cosmetic, pharmaceutical, soil, water, drinking water and/or wastewater samples.

## Revendications

**1.** Procédé pour la détection de composés à action hormonale, comprenant les étapes consistant à :

a) fournir une surface comportant un partenaire de liaison à l'analyte immobilisé,
dans lequel le partenaire de liaison à l'analyte comprend une sulfotransférase choisie parmi l'oestrogène sulfotransférase hSULT1E1 (oestrogène sulfotransférase humaine) ou une oestrogène sulfotransférase homologue,
b) mettre en contact le partenaire de liaison à l'analyte avec un échantillon contenant l'analyte,
dans lequel l'analyte est un composé à action hormonale comprenant au moins un groupe phénol et interagit avec le partenaire de liaison à l'analyte,
c) mettre en contact le partenaire de liaison à l'analyte avec un compétiteur,

dans lequel le compétiteur est un composé à action hormonale comprenant au moins un groupe phénol et est lié à une particule déformable par l'intermédiaire d'un groupe fonctionnel,
dans lequel la particule déformable est une particule d'hydrogel et/ou présente un module d'élasticité dans la gamme allant de 5 kPa à 100 kPa,
dans lequel le compétiteur interagit avec le partenaire de liaison à l'analyte,

d) détecter les compétiteurs liés au partenaire de liaison à l'analyte par détection de la déformation des particules d'hydrogel.

**2.** Procédé selon la revendication 1, **caractérisé en ce que** l'œstrogène sulfotransférase homologue présente une identité de séquence d'au moins 85 % avec la hSULT1E1 selon SEQ ID NO : 1.

**3.** Procédé selon la revendication 1 ou 2, **caractérisé en ce que** l'analyte est un composé de formule (I) ou (II)

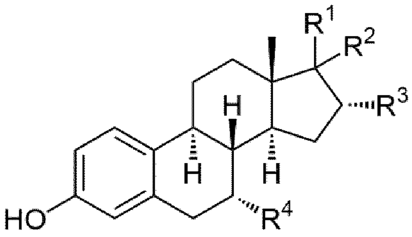 (I) 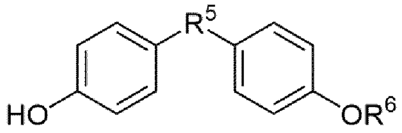 (II),

dans lequel $R^1$, $R^2$, $R^3$, $R^4$, $R^6$ sont choisis respectivement indépendamment les uns des autres parmi H, groupes fonctionnels, groupes alkyle, alcényle ou aryle en C1 à C15 non substitués ou substitués, ou groupes glucidiques,
dans lequel $R^5$ est choisi parmi des groupes alkyle, alcényle ou aryle en C1 à C15, non substitués ou substitués, ou des groupes glucidiques.

4. Procédé selon l'une des revendications 1 à 3, **caractérisé en ce que** l'analyte est choisi parmi les oestrogènes, dérivés d'oestrogènes, hydrocarbures aromatiques halogénés hydroxylés, nonylphénols, bisphénol A, anti-oestrogènes phénoliques et phytoaestrogènes phénoliques.

5. Procédé selon l'une des revendications 1 à 4, **caractérisé en ce que** le partenaire de liaison à l'analyte est une protéine de fusion, de préférence une protéine de fusion comportant une étiquette de protéine ou une hydrophobine.

6. Procédé selon l'une des revendications précédentes, **caractérisé en ce que** le compétiteur est un composé de formule (I) ou (II)

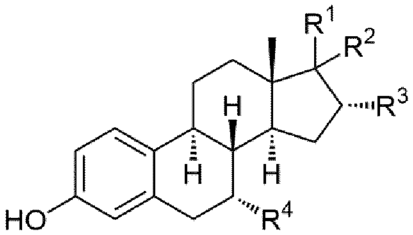 (I) 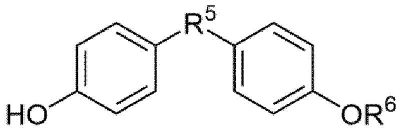 (II),

dans lequel $R^1$, $R^2$, $R^3$, $R^4$, $R^6$ sont choisis respectivement indépendamment les uns des autres parmi H, groupes fonctionnels, groupes alkyle, alcényle ou aryle en C1 à C15 non substitués ou substitués, ou groupes glucidiques,
dans lequel $R^5$ est choisi parmi des groupes alkyle, alcényle ou aryle en C1 à C15, non substitués ou substitués, ou des groupes glucidiques,
dans lequel le compétiteur présente un groupe fonctionnel en position $R^1$, $R^2$ ou $R^6$, par l'intermédiaire duquel le compétiteur est lié à la particule déformable.

7. Procédé selon l'une des revendications précédentes, **caractérisé en ce que** le compétiteur est le β-estradiol-17-(β-D-glucuronide).

8. Procédé selon l'une des revendications précédentes, **caractérisé en ce que** le compétiteur est lié à la particule déformable par l'intermédiaire d'un lieur, dans lequel le lieur présente une longueur de contour dans la gamme allant de 5 Å à 200 Å et/ou un degré de polymérisation dans la gamme allant de 1 à 70.

9. Procédé selon l'une des revendications précédentes, **caractérisé en ce que** la détection est effectuée par microscopie à contraste interférentiel par réflexion.

10. Procédé selon l'une des revendications précédentes, **caractérisé en ce que** la surface est conçue de manière à être transparente au moins dans la gamme allant de 400 nm à 600 nm.

11. Kit comprenant :

i. une surface comportant un partenaire de liaison à l'analyte immobilisé,

dans lequel le partenaire de liaison à l'analyte comprend une sulfotransférase choisie parmi l'œstrogène sulfotransférase hSULT1E1 (oestrogène sulfotransférase humaine) ou une oestrogène sulfotransférase homologue,

ii. au moins une particule déformable présentant un compétiteur immobilisé,

dans lequel la particule déformable est une particule d'hydrogel et/ou présente un module d'élasticité dans la gamme allant de 5 kPa à 100 kPa,
dans lequel le compétiteur est un composé à action hormonale comprenant au moins un groupe phénol et est lié à la particule déformable par l'intermédiaire d'un groupe fonctionnel,
dans lequel le compétiteur interagit avec le partenaire de liaison à l'analyte.

**12.** Kit selon la revendication 11, **caractérisé en ce que** l'oestrogène sulfotransférase homologue présente une identité de séquence d'au moins 85 % avec la hSULT1E1 selon SEQ ID NO : 1.

**13.** Kit selon la revendication 11 ou 12, **caractérisé en ce que** le compétiteur est un composé de formule (I) ou (II)

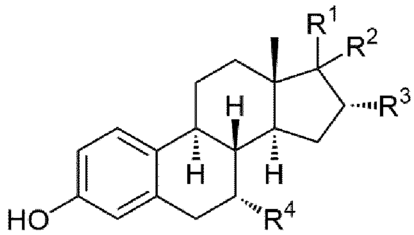 (I) 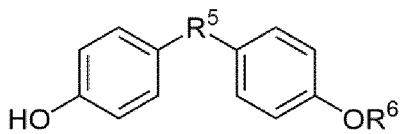 (II),

dans lequel $R^1$, $R^2$, $R^3$, $R^4$, $R^6$ sont choisis respectivement indépendamment les uns des autres parmi H, groupes fonctionnels, groupes alkyle, alcényle ou aryle en C1 à C15 non substitués ou substitués, ou groupes glucidiques,
dans lequel $R^5$ est choisi parmi des groupes alkyle, alcényle ou aryle en C1 à C15, non substitués ou substitués, ou des groupes glucidiques,
dans lequel le compétiteur présente un groupe fonctionnel en position $R^1$, $R^2$ ou $R^6$, par l'intermédiaire duquel le compétiteur est lié à la particule déformable.

**14.** Utilisation d'un procédé selon l'une des revendications 1 à 10 et/ou d'un kit selon l'une des revendications 11 à 13 pour la détection de composés à action hormonale dans des échantillons d'aliments, d'agents de lavage, d'agents de rinçage, de produits de soin du corps, de cosmétiques, de produits pharmaceutiques, de sol, d'eau, d'eau potable et/ou d'eaux usées.

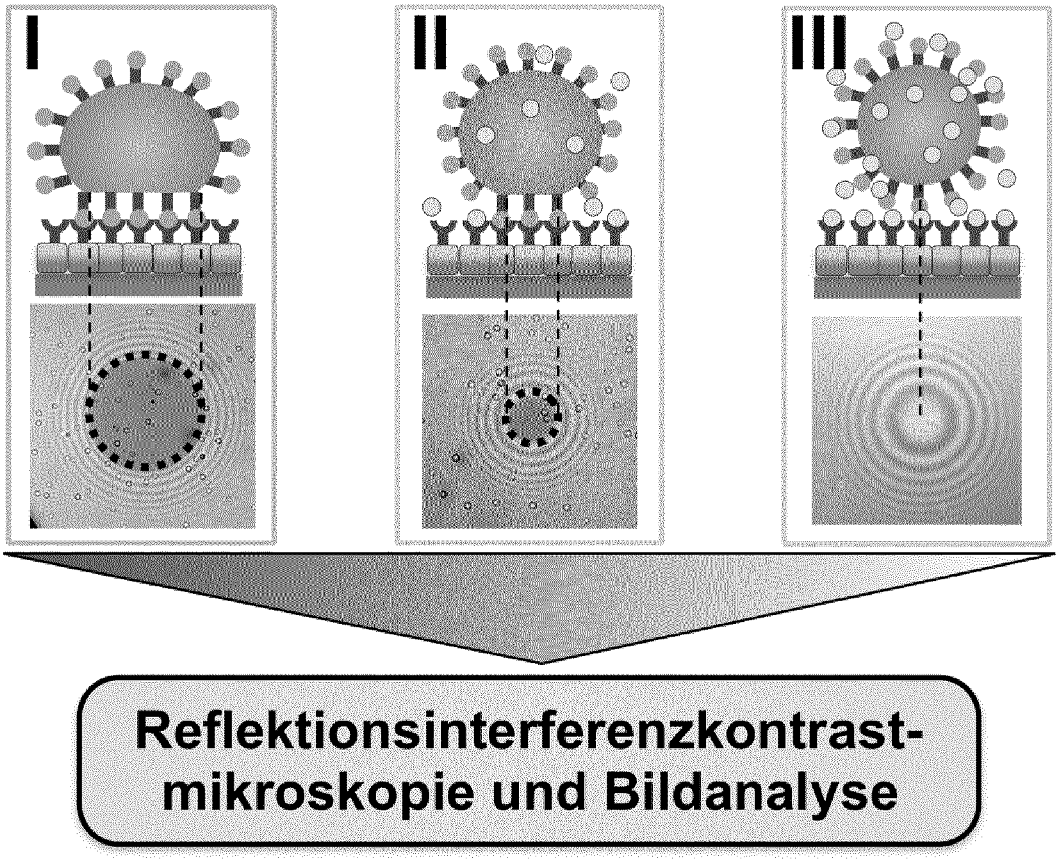
I
II
III
Reflektionsinterferenzkontrast-
mikroskopie und Bildanalyse

Fig. 1

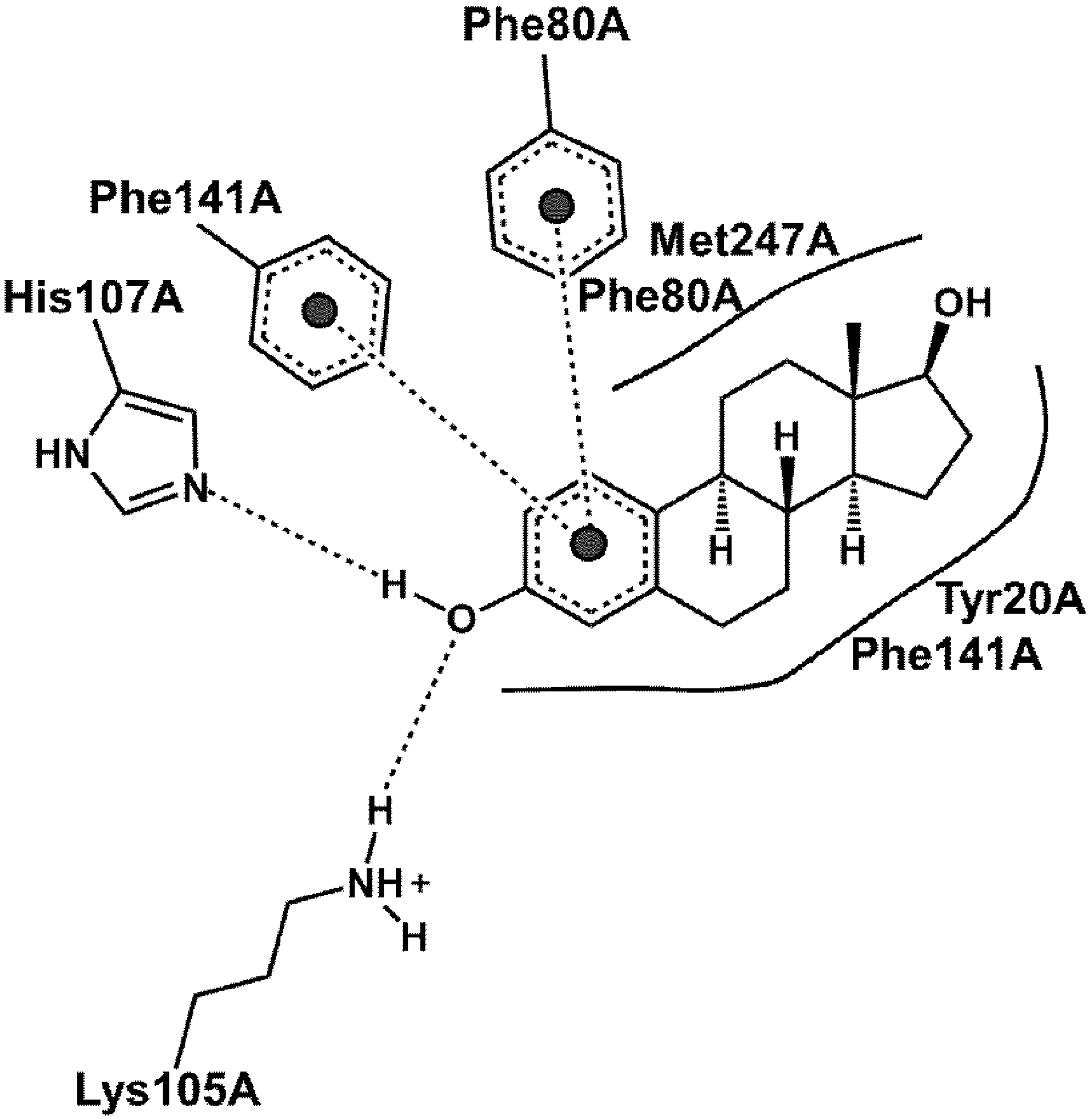
Phe80A
Phe141A
His107A
Met247A
Phe80A
Tyr20A
Phe141A
Lys105A

Fig. 2A

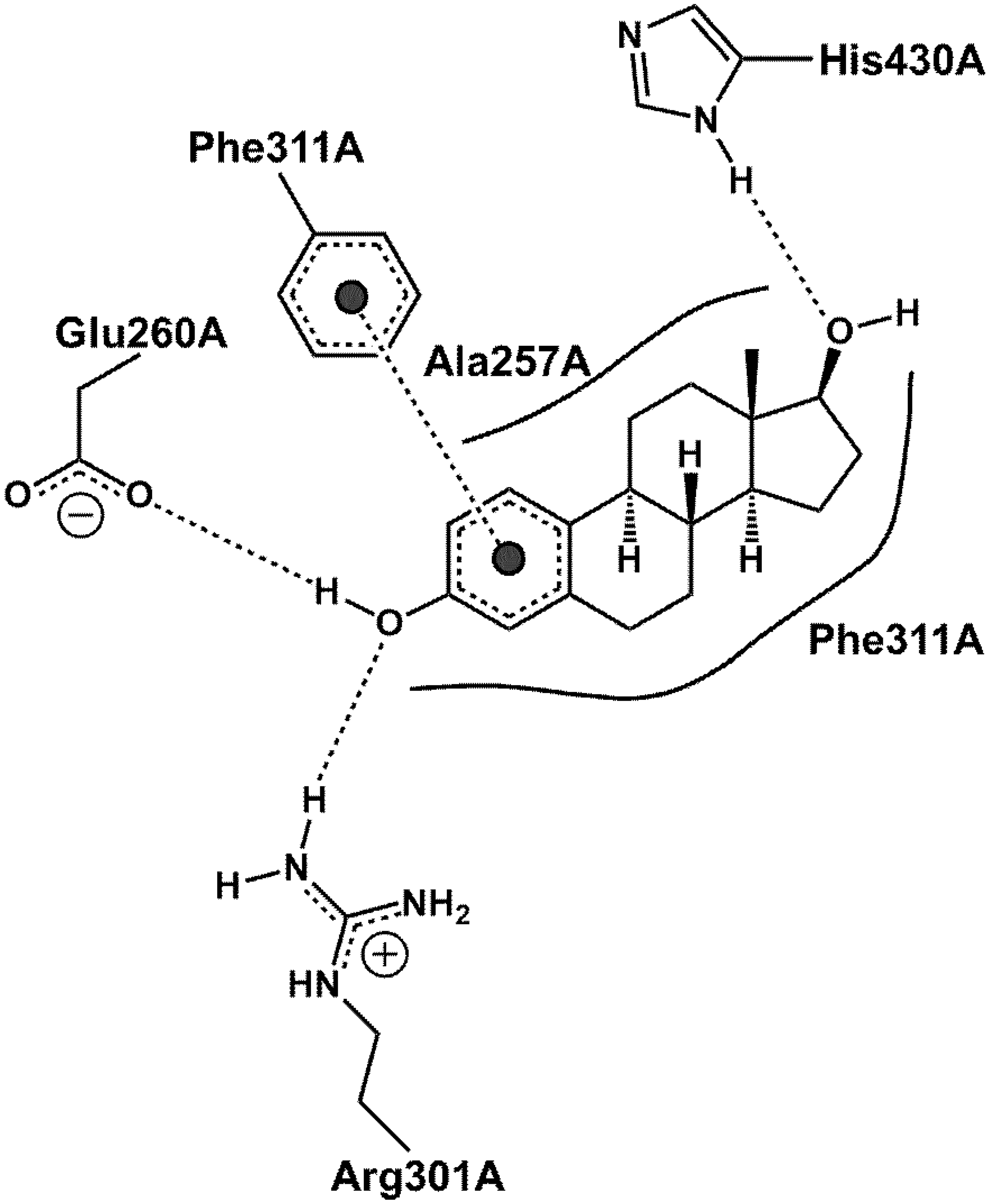

Fig. 2B

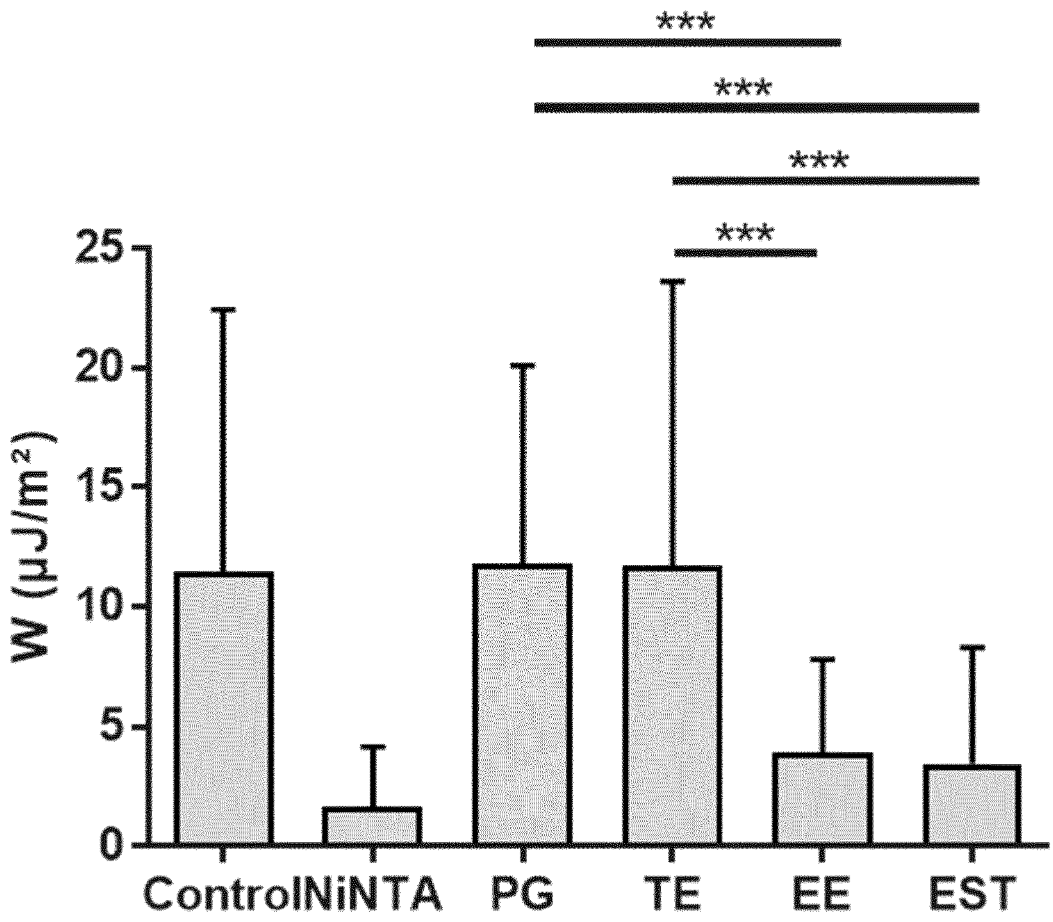


Fig. 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2014106665 A1
- WO 02053713 A2
- US 7288641 B1


### In der Beschreibung aufgeführte Nicht-Patentliteratur


- **BECK V ; PFITSCHER A ; JUNGBAUER A.** GFP-reporter for a high throughput assay to monitor estrogenic compounds. *J Biochem Biophys Methods,* 2005, vol. 64 (1), 19-37
- **BITTNER M ; JARQUE S ; HILSCHEROVA K.** Polymer-immobilized ready-to-use recombinant yeast assays for the detection of endocrine disruptive compounds. *Chemosphere,* 2015, vol. 132, 56-62
- **BOVEE TFH ; HELSDINGEN RJR ; HAMERS ARM ; VAN DUURSEN MBM ; NIELEN MWF ; HOOGENBOOM RLAP.** A new highly specific and robust yeast androgen bioassay for the detection of agonists and antagonists. *Anal Bioanal Chem,* 2007, vol. 389 (5), 1549-1558
- **COLE GB ; KEUM G ; LIU J ; SMALL GW ; SATAYAMURTHY N ; KEEP V ; BARRIO JR.** Specific estrogen sulfotransferase (SULT1E1) Substrates and molecular imaging probe candidates. *Proceedings of the National Academy of Sciences,* 2010, vol. 107 (14), 6222-6227
- **DU L ; JI W ; ZHANG Y ; ZHANG C ; LIU G ; WANG S.** An ultrasensitive detection of 17β-estradiol using a gold nano-particle-based fluorescence immunoassay. *Analyst,* 2015, vol. 140 (6), 2001-2007
- Executive summary. *EFSA Journal,* vol. 13 (1), 3978
- **JOHNSON KL ; KENDALL K ; ROBERTS AD.** Surface energy and the contact of elastic solids. *Proc. R. Soc. Lond. Ser. A - Math. Phys. Sci.,* 1971, vol. 324, 301
- **KESTER MHA ; BULDUK S ; VAN TOOR H ; TIBBOEL D ; MEINL W ; GLATT H ; FALANY CN ; COUGHTRIE MWH ; SCHUUR AG ; BROUWER A.** Potent Inhibition of Estrogen Sulfotransferase by Hydroxylated Metabolites of Polyhalogenated Aromatic Hydrocarbons Reveals Alternative Mechanism for Estrogenic Activity of Endocrine Disrupters. *The Journal of Clinical Endocrinology & Metabolism,* 2002, vol. 87 (3), 1142-1150
- **LIU L ; ZHOU X ; LU Y ; SHAN D ; XU B ; HE M ; SHI H ; QIAN Y.** Facile screening of potential xenoestrogens by an estrogen receptor-based reusable optical biosensor. *Biosensors and Bioelectronics,* 2017, vol. 97, 16-20
- **PURVIS IJ ; CHOTAI D ; DYKES CW ; LUBAHN DB ; FRENCH FS ; WILSON EM ; HOBDEN AN.** An androgen-inducible expression system for Saccharomyces cerevisiae. *Gene,* 1991, vol. 106, 35-42
- **PUSSAK D ; BEHRA M ; SCHMIDT S ; HARTMANN L.** *Soft Matter,* 2012, vol. 8, 1664
- **PUSSAK D ; PONADER D ; MOSCA S ; POMPE T ; HARTMANN L ; SCHMIDT S.** Specific Adhesion of Carbohydrate Hydrogel Particles in Competition with Multivalent Inhibitors Evaluated by AFM. *Langmuir,* 2014, vol. 30 (21), 6142-6150
- **ROUTLEDGE EJ ; SUMPTER JP.** Estrogenic activity of surfactants and some of their degradation products assessed using a recombinant yeast screen. *Environmental Toxicology and Chemistry,* 1996, vol. 15 (3), 241-248
- **SOHONI P ; SUMPTER JP.** Several environmental oestrogens are also anti-androgens. *Endocrinol,* 1998, vol. 158, 327-339
- **STJERNSCHANTZ E ; REINEN J ; MEINL W ; GEORGE BJ ; GLATT H ; VERMEULEN NPE ; OOSTENBRINK C.** Comparison of murine and human estrogen sulfotransferase inhibition in vitro and in silico - Implications for differences in activity, subunit dimerization and substrate inhibition. *Mol. Cell. Endocrinol.,* 2010, vol. 317, 127-140
- **WOLF S ; RATAJ F ; ZIERAU O ; OSTERMANN K ; DIEL P ; PARR MK ; VOLLMER G ; RÖDEL G.** A novel combined approach to detect androgenic activities with yeast based assays in Schizosaccharomyces pombe and Saccharomyces cerevisiae. *Toxicology Letters,* 2010, vol. 199, 410-415